# EUROPEAN PATENT SPECIFICATION

(11) **EP 0 518 238 B1**
(45) Date of publication and mention of the grant of the patent: **07.04.1999**
(21) Application number: 92109588.1
(22) Date of filing: 05.06.1992
(51) Int. Cl.: C07D 487/04

(54) **Pyrrolotriazole derivative**
Pyrrolotriazolderivat
Dérivé de pyrrolotriazole

(30) Priority: 07.06.1991 JP 162324/91; 27.11.1991 JP 311212/91; 27.11.1991 JP 335861/91; 21.02.1992 JP 69980/92
(43) Date of publication of application: 16.12.1992
(60) Divisional application: 98117148.1
(73) Proprietor: FUJI PHOTO FILM CO., LTD., Kanagawa-ken (JP)
(72) Inventor: Suzuki, Makoto, c/o Fuji Photo Film Co., Ltd., Minami, Ashigara-shi, Kanagawa (JP); Mikoshiba, Hisashi, c/o Fuji Photo Film Co., Ltd., Minami, Ashigara-shi, Kanagawa (JP); Takahashi, Osamu, c/o Fuji Photo Film Co., Ltd., Minami, Ashigara-shi, Kanagawa (JP); Shimada, Yasuhiro, c/o Fuji Photo Film Co., Ltd., Minami, Ashigara-shi, Kanagawa (JP); Matsuoka, Koushin, c/o Fuji Photo Film Co., Ltd., Minami, Ashigara-shi, Kanagawa (JP); Yamazaki, Shigeru, c/o Fuji Photo Film Co., Ltd., Minami, Ashigara-shi, Kanagawa (JP); Yamakawa, Kazuyoshi, c/o Fuji Photo Film Co., Ltd., Minami, Ashigara-shi, Kanagawa (JP); Sato, Kozo, c/o Fuji Photo Film Co., Ltd., Minami, Ashigara-shi, Kanagawa (JP)
(74) Representative: Grünecker, Kinkeldey, Stockmair & Schwanhäusser Anwaltssozietät

(56) References cited:
- EP-A- 252 288
- US-A- 4 093 728
- PATENT ABSTRACTS OF JAPAN vol. 12, no. 180 (P-709)10 June 1986 & JP-A-62 289 837 ( KONISHIROKU SHASHIN KOGYO K.K. ) 16 December 1987

## Description

The present invention relates to a novel pyrrolotriazole derivative

1H-Pyrrolo-[1,2-b][1,2,4]triazole derivaties are useful as synthesis intermediates of physiologically active substances such as pharmaceutical preparations and pesticides. These derivatives are also known as coupler nuclei and dyes which exhibit reduced secondary absorption in the field of photographic chemistry (proceedings of the 60th Annual Conference of The Society of Photographic Science and Technology of Japan). However, 1H-pyrrolo-[1,2-b][1,2,4]triazole dyes which have heretofore been known exhibit a maximum absorption wavelength of lower than 560 nm. No 1H-pyrrolo-[1,2-b][1,2,4]triazole dyes which exhibit a maximum absorption wavelength of higher than 600 nm have been known.

In recent years, 1H-pyrrolo-[1,2-b][1,2,4]triazole dyes which exhibit a maximum absorption wavelength of higher than 560 nm, particularly 600 nm have been desired. In other words, dyes containing a 1H-pyrrolo-[1,2-b][1,2,4]triazole nucleus which exhibit reduced secondary absorption and a primary absorption wavelength of higher than 600 nm have been keenly desired.

Furthermore, in recent years, new color image formation methods such as color electrophotography, ink jet printing process and heat-sensitive transfer process have been proposed. On the other hand, with the development of electronic imaging technique, the demand for solid state image pick-up tube and filter for color liquid crystal television set has increased. Thus, azomethine dyes have been applied and reviewed in color photography as well as various systems or merchandise.

As cyan azomethine dyes in these applications there have been known phenol and naphthol azomethine dyes. Furthermore, imidazole azomethine dyes and hydroxypyridine azomethine dyes have been known.

Moreover, pyrazoloazole azomethine dyes, pyrazolopyrimidin-5-one azomethine dyes, pyrazoloquinazolone azomethine dyes, pyrazolotriazine azomethine dyes and cyan azomethine dyes have been known.

However, these known azomethine dyes have various disadvantages. For example, phenol and naphthol azomethine dyes exhibit too broad an absorption to serve as dyes for filter. Further, imidazole azomethine dyes are disadvantageous in that they exhibit a low fastness to light. Moreover, pyrazolopyrimidin-5-one azomethine dyes, pyrazoloquinazolone azomethine dyes and pyrazolotriazine azomethine dyes exhibit too broad an absorption. Further, pyrazolotriazole azomethine dyes exhibit a low fastness to light. Moreover, hydroxypyridine azomethine dyes can hardly be synthesized and exhibit a low fastness to light.

Thus, among known azomethine dyes, there are no dyes which exhibit an absorption waveform suitable for cyan color and a high fastness to light and heat. Therefore, the development of azomethine dyes which exhibit a sharp absorption and a high fastness has been keenly desirerd.

In order to overcome these difficulties, the inventors made a study on novel azomethine dyes. As a result, it was found that pyrrolotriazole azomethine dyes having a specific structure which has never been known exhibit a sharp absorption and a high fastness to light. Thus, the present invention was worked out.

Pyrrolotriazole couplers are disclosed in JP-A-62-279340 and 62-278552 (the term "JP-A" as used herein means an "unexamined published Japanese patent application").

Even if the foregoing techniques are known, the present invention is by no means limited thereby.

The above mentioned known couplers are considered to undergo color development to produce azomethine dyes. According to the above cited references, the color of the dyes thus produced are magenta.

In the above cited references, there is no reference to couplers which will give an azomethine dye that exhibits a cyan color. There is neither reference that gives an expectation of a specific structure that exhibits a cyan color.

It is therefore the object of the present invention to provide a novel pyrrolotriazole derivative which exhibits a sharp absorption and a high fastness to heat and light.

The object of the present invention is achieved with pyrrolotriazole derivatives represented by formulae (I) to (III) 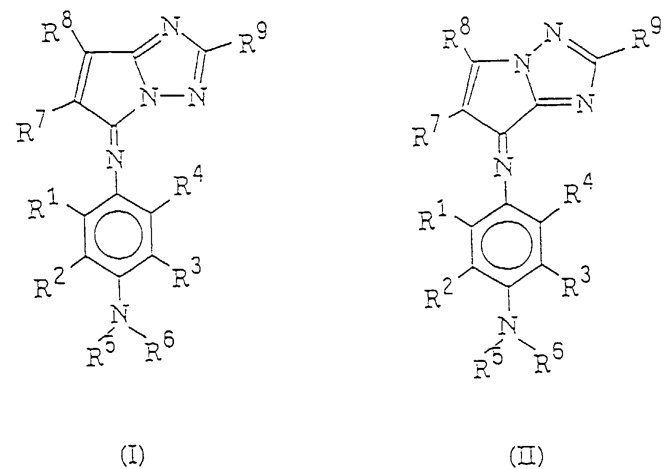 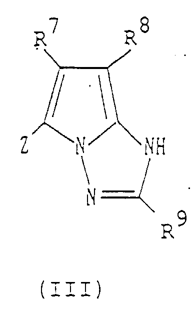 wherein:
R¹, R², R³, and R⁴ each independently represents a hydrogen atom, an alkyl group, an alkoxy group or an acylamino group;
R⁵ and R⁶ each independently represents a hydrogen atom, an alkyl group, an aryl group, or a heterocyclic group;
R⁷ represents a cyano group, an alkoxycarbonyl group, a trifluoromethyl group, or a carbamoyl group;
R⁸ represents a cyano group, a sulfonyl group, a carbamoyl group, an alkoxycarbonyl group, a trifluoromethyl group or an aryloxycarbonyl group;
R9 represents a hydrogen atom, an unsubstituted aryl group or an aryl group substituted by at least one of substituents selected from a nitro group, a halogen atom, a cyano group, an acylamino group, and a sulfonamido group, a heterocyclic group, an alkyl group, a cyano group, a carboxyl group, a formyl group, an acyl group, a carbamoyl group, an alkoxycarbonyl group, an aryloxycarbonyl group, an acylamino group, an alkoxycarbonylamino group, an aryloxycarbonylamino group, a sulfonylamino group, an aminocarbonylamino group, a sulfamoylamino group, an amino group, an alkoxy group, an aryloxy group, a silyloxy group, a heteryloxy group, an alkylthio group, an arylthio group, a heterylthio group, a halogen atom, a hydroxyl group, a nitro group, a sulfamoyl group, a sulfonyl group, an azo group, an acyloxy group, a carbamoyloxy group, an imido group, a sulfinyl group, a phosphoryl group, or an azolyl group;
the sum of Hammett's substituents constant σₚ of R⁷ and R⁸ is 0.65 or more;
and
Z represents a hydrogen atom, a halogen atom, an arylthio group, a heterocyclic thio group, an arylsulfinyl group, or a nitroso group, wherein the arylthiogroup is an unsubstituted arylthio group or an arylthio group substituted by at least one of substituents selected from a halogen atom, an alkyl group, an alkoxy group, a cyano group, an aryloxycarbonyl group, an alkoxycarbonyl group, a ureido group, an alkoxycarbonylamino group, an acylamino group and a carboxyl group, and the arylsulfinyl group is an unsubstituted aryl sulfinyl group or an aryl sulfinyl group substituted by at least one of substituents selected from a halogen atom, an alkyl group, an alkoxy group, a cyano group, an alkoxycarbonyl group and a carboxyl group.

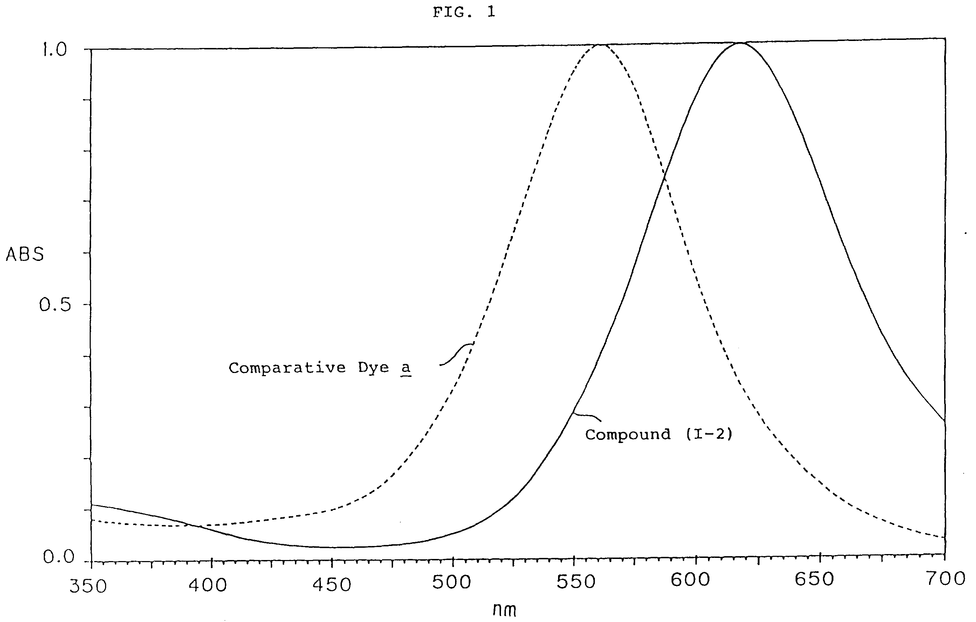

Fig. 1 shows the absoption spectrum of ethyl acetate solution of exemplary Compound (I-2) and a comparative dye a.

Among the compounds represented by formulae (I) to (III), those represented by formulae (I) and (II) are useful as cyan dyes.

Further, the 1H-pyrrolo-[1,2-b][1,2,4]triazole derivatives represented by formula (III) are useful as synthesis intermediates of the compounds represented by formulae (I) and (II), or physiologically active substances such as pharmaceutical preparations and pesticides.

Formulae (I) to (III) will be further described hereinafter.

Hammett's substituent constant as used herein will be now briefly discussed. Hammett's rule is an empirical law which was proposed in 1935 by L. P. Hammett to give a quantative discussion of the effect of substituents on the reaction or equilibrium of benzene derivatives. This rule is now widely considered reasonable. Substituent constants determined by Hammett's rule include σ_{P} value and σₘ value which can be found in many general references, e.g., J.A. Dean, Lange's Handbook of Chemistry, vol. 12, 1979 (McGraw-Hill), and Kagaku No Ryoiki (The Domain of Chemistry), extra edition, No. 122, pp. 96 - 103, 1979 (Nankodo). In the present invention, various substituents are limited or illustrated by Hammett's substituent constant σ_{P}. However, this doesn't mean that these substituents are limited to those having known σ_{P} values found in the above cited references. It is clear that even when these substituents exhibit σ_{P} values unknown in any reference, they are included among those having σ_{P} values that would be included in the range known in these references when measured according to Hammett's rule. The value of σ_{P} will be hereinafter defined in this manner.

R¹, R², R³ and R⁴ each independently represents a hydrogen atom, an alkyl group, an alkoxy group or an acylamino group.

Specific examples of R¹, R², R³ an R⁴ include hydrogen atom, alkyl group (preferably alkyl group having a total carbon atom number of from 1 to 30 (hereinafter expressed as "C₁₋₃₀ alkyl group"), e.g., methyl, ethyl, propyl, butyl), alkoxy group (preferably C₁₋₃₀ alkoxy group, e.g., methoxy, ethoxy, methoxyethoxy, isopropoxy), acylamino group (preferably C₁₋₃₀ alkylcarbonylamino group, e.g., formylamino, acetylamino, propionylamino, cyanoacetyl amino , or preferably C₇₋₃₀ arylcarbonylamino group, e.g., benzoylamino, p-toluylamino, pentafluorobenzoylamino, m-methoxybenzoylamino).

Preferred among the groups represented by R², R³ and R⁴ is hydrogen atom.

Preferred among the groups represented by R¹ are hydrogen atom, C₁₋₃₀ alkyl group, C₁₋₃₀ alkoxy group, and, C₁₋₃₀ acylamino group. Particularly preferred among these groups are hydrogen atom, alkyl group, and acylamino group.

R⁵ and R⁶ each independently represents a hydrogen atom, alkyl group, aryl group, or heterocyclic group.

Specific examples of R⁵ and R⁶ include a hydrogen atom, alkyl group (preferably C₁₋₃₀ alkyl group, e.g., methyl, ethyl, propyl, isopropyl, butyl, octyl, aryl group (preferably C₆₋₃₀ aryl group, e.g., phenyl, p-tolyl, 2-naphthyl) or heterocyclic groups preferably C₃₋₃₀ heterocyclic group such as those represented by the following formulae:

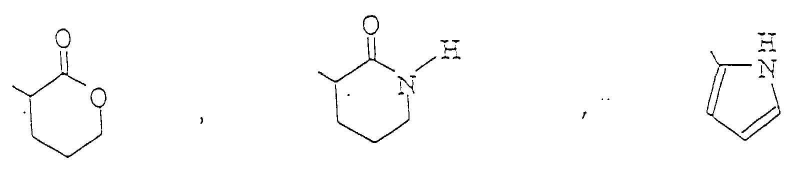

Preferred among the groups represented by R⁵ and R⁶ is a C₁₋₃₀ alkyl group (e.g., methyl, ethyl, propyl). Examples of R⁷ include alkoxycarbonyl group, cyano group.

Specific examples of R⁷ include carbamoyl group (e.g., N-ethylcarbamoyl, N,N-dibutylcarbamoyl, N-(2-dodecyloxyethyl)-carbamoyl, N-methyl-N-dodecylcarbamoyl, N-[3-(2,4-di-t-amylphenoxy)propyl]carbamoyl), alkoxycarbonyl group (preferably straight-chain, branched or cyclic alkoxycarbonyl group, e.g., methoxycarbonyl, ethoxycarbonyl, isopropoxycarbonyl, isobutoxycarbonyl, n-octyloxycarbonyl, n-decyloxycarbonyl, n-dodecyloxycarbonyl, n-hexadecyloxycarbonyl, 2-ethylhexyloxycarbonyl, 3,5,5-trimethylhexyloxycarbonyl, 2-ethyl-4-methylpentyloxycarbonyl, 2-hexyldecyloxycarbonyl, 2-heptylundecyloxycarbonyl, 2-octyldodecyloxycarbonyl, 2,4,6-tri-methylheptyloxycarbonyl, 2,4,6,8-tetramethylnonyloxycarbonyl, benzyloxycarbonyl, 2-phenethyloxycarbonyl, 3-(t-octylphenoxy)-propoxycarbonyl, 3-(2,4-di-t-pentylphenoxy)propoxycarbonyl, 2-(4-biphenyloxy)ethoxycarbonyl, 3-dodecyloxypropoxycarbonyl, 2-dodecylthioethoxycarbonyl 9,10-epoxyoctadecyloxycarbonyl, dodecyloxycarbonylmethoxycarbonyl, 2-(2-naphthyloxy)ethoxycarbonyl, 7,7-dimethyl-2-(3',3'-dimethylbutyl)-5-methyloctyloxycarbonyl, 2-methyl-cyclohexyloxycarbonyl, 2-hexyl-cyclohexyloxycarbonyl), cyano group, and a trifluoromethyl group.

Examples of σ values of typical groups include 0.66 for cyano group, 0.54 for trifluoromethyl group, 0.36 for carbamoyl group, 0.45 for methoxycarbonyl group.

Preferred among the groups represented by R⁷ are carbamoyl group, alkoxycarbonyl group, cyano group.

Particularly preferred among these groups are cyano group and alkoxycarbonyl group.

That is, R⁷ is preferably a cyano group or -COR wherein R is preferably C₁₋₃₆, preferably C₁₋₂₄ straight-chain, branched or cyclic alkoxy group.

Alternatively, R⁷ is preferably a cyano group or -COR wherein R is preferably an straight-chain, branched or cyclic alkoxy group (e.g., methoxy, ethoxy, isopropoxy, isobutoxy, n-octyloxy, n-decyloxy, n-dodecyloxy, n-hexadecyloxy, 2-ethylhexyloxy, 3,5,5-trimethylhexyloxy, 2-ethyl-4-methylpentyloxy, 2-hexyldecyloxy, 2-heptylundecyloxy, 2-octyldodecyloxy, 2,4,6-trimethylheptyloxy, 2,4,6,8-tetramethylnonyloxy).

In particular R⁹ represents a hydrogen atom, an aryl group, a heterocyclic group, an alkyl group, a cyano group, a carboxyl group, a formyl group, an acyl group, a carbamoyl group, an alkoxycarbonyl group, an aryloxycarbonyl group, an acylamino group, an alkoxycarbonylamino group, an aryloxycarbonylamino group, a sulfonylamino group, an aminocarbonylamino group, a sulfamoylamino group, an amino group (including an anilino group), an alkoxy group, an aryloxy group, a silyloxy group, a heteryloxy group, an alkylthio group, an arylthio group, a heterylthio group, a halogen atom, a hydroxyl group, a nitro group, a sulfamoyl group, a sulfonyl group, an azo group, an acyloxy group, a carbamoyloxy group, an imido group, a sulfinyl group, a phosphoryl group or an azolyl group.

Specific examples of R⁹ include a hydrogen atom, an aryl group (preferably a C₆₋₃₀ aryl group, e.g., phenyl, m-acetylaminophenyl, p-methoxyphenyl), an alkyl group (preferably a C₁₋₃₀ alkyl group, e.g., methyl, ethyl, isopropyl, t-butyl, n-octyl, n-dodecyl), a cyano group, a carboxyl group, an acyl group (preferably a C₁₋₃₀ acyl group, e.g., acetyl, pivaloyl, benzoyl, furoil, 2-pyridylcarbonyl), a carbamoyl group (preferably a C₁₋₃₀ carbamoyl group, e.g., methylcarbamoyl, ethylcarbamoyl, dimethylcarbamoyl, n-octylcarbamoyl), an alkoxycarbonyl group (preferably a C₁₋₃₀ alkoxycarbonyl group, e.g., methoxycarbonyl, ethoxycarbonyl, isopropoxycarbonyl), an aryloxycarbonyl group (preferably a C₇₋₃₀ aryl group, e.g., phenoxycarbonyl, p-methoxyphenoxycarbonyl, m-chlorophenoxycarbonyl, o-methoxyphenoxycarbonyl), an acylamino group (preferably a C₁₋₃₀ alkylcarbonylamino group, e.g., formylamino, acetylamino, propionylamino, cyanoacetylamino, C₇₋₃₀ arylcarbonylamino group, e.g., benzoylamino, p-toluylamino, pentafluorobenzoylamino, m-methoxybenzoylamino, or C₄₋₃₀ heterylcarbonylamino group, e.g., 2-pyridylcarbonylamino, 3-pyridylcarbonylamino, furoylamino), an alkoxycarbonylamino group (preferably a C₂₋₃₀ alkoxycarbonylamino group, e.g., methoxycarbonylamino, ethoxycarbonylamino, methoxyethoxycarbonylamino), an aryloxycarbonylamino group (preferably a C₇₋₃₀ aryloxycarbonylamino group, e.g., phenoxycarbonylamino, p-methoxyphenoxycarbonylamino, p-methylphenoxycarbonylamino, m-chlorophenoxycarbonylamino, o-chlorophenoxycarbonylamino), a sulfonylamino group (preferably a C₁₋₃₀ sulfonylamino group, e.g., methanesulfonylamino, benzenesulfonylamino, toluenesulfonylamino), an aminocarbonylamino group (preferably a C₁₋₃₀ aminocarbonylamino group, e.g., methylaminocarbonylamino, ethylaminocarbonyl amino, anilinocarbonylamino, dimethylaminocarbonylamino), a sulfamoylamino group (preferably a C₁₋₃₀ sulfamoylamino group, e.g., methylaminosulfonylamino, ethylaminosulfonylamino, anilinosulfonylamino), an amino group (including an anilino group, preferably a C₀₋₃₀ amino group, e.g., amino, methylamino, dimethylamino, ethylamino, diethylamino, n-butylamino, anilino), an alkoxy group (preferably a C₁₋₃₀ alkoxy group, e.g., methoxy, ethoxy, isopropoxy, n-butoxy, methoxyethoxy, n-dodecyloxy), an aryloxy group (preferably a C₆₋₃₀ aryloxy group, e.g., phenoxy, m-chlorophenoxy, p-methoxyphenoxy, o-methoxyphenoxy), a silyloxy group (preferably a C₃₋₃₀ silyloxy group, e.g., trimethylsilyloxy, t-butyldimethylsilyloxy, cetyldimethylsilyloxy, phenyldimethylsilyloxy), a heteryloxy group (preferably a C₃₋₃₀ heteryloxy group, e.g., tetrahydropyranyloxy, 3-pyridyloxy, 2-(1,3-benzimidazolyl)oxy), an alkylthio group (preferably a C₁₋₃₀ alkylthio group, e.g., methylthio, ethylthio, n-butylthio, t-butylthio), an arylthio group (preferably a C₆₋₃₀ arylthio group, e.g., phenylthio), a heterylthio group (preferably a C₃₋₃₀ heterylthio group, e.g., 2-pyridylthio, 2-(1,3-benzoxazolyl)thio, 1-hexadecyl-1,2,3,4-tetrazolyl-5-thio, 1-(3-N-octadecylcarbamoyl)phenyl-1,2,3,4-tetrazolyl-5-thio), a heterocyclic group (preferably a C₃₋₃₀ heterocyclic group, e.g., 2-benzoxazolyl, 2-benzothiazolyl, 1-phenyl-2-benzimidazolyl, 5-chloro-1-tetrazolyl, 1-pyrrolyl, 2-furanyl, 2-pyridyl, 3-pyridyl), a halogen atom (e.g., fluorine, chlorine, bromine), a hydroxyl group, a nitro group, a sulfamoyl group (preferably a C₀₋₃₀ sulfamoyl group, e.g., methylsulfamoyl, dimethylsulfamoyl), a sulfonyl group (preferably a C₁₋₃₀ sulfonyl group, e.g., methanesulfonyl, benzenesulfonyl, toluenesulfonyl), an azo group (preferably a C₃₋₃₀ azo group, e.g., p-nitrophenylazo), an acyloxy group (preferably C₁₋₃₀ acyloxy group, e.g., formyloxy, acetyloxy, benzoyloxy), a carbamoyloxy group (preferably a C₁₋₃₀ aryl group, e.g., methylcarbamoyloxy, diethylcarbamoyloxy), an imido group (preferably a C₄₋₃₀ imido group, e.g., succinimido, phthalimido), a sulfinyl group (preferably a C₁₋₃₀ sulfinyl group, e.g., diethylaminosulfinyl), a phosphoryl group (preferably a C₀₋₃₀ phosphoryl group, e.g., diaminophosphoryl), and an azolyl group (preferably a C₂₋₃₀ azolyl group, e.g., 3-pyrazolyl).

Particularly preferred examples of R⁸ include cyano group, carbamoyl group, alkoxycarbonyl group, and aryloxycarbonyl group. That is, R⁸ is preferably a cyano group or -COR wherein R is an amino group, an alkoxy group or an aryloxy group. More preferred among these groups are cyano group, alkoxycarbonyl group, and aryloxycarbonyl group. Particularly preferred among these groups are alkoxycarbonyl group and aryloxycarbonyl group.

In the present invention, the sum of σ_{P} values of R⁷ and R⁸ is 0.65 or more. Examples of such a combination will be set forth below, but the present invention should not be construed as being limited thereto.

| R⁷ | | R⁸ | | Sum |
|---|---|---|---|---|
| CN | 0.66 | CN | 0.66 | 1.32 |
| CN | 0.66 | CO₂CH₃ | 0.45 | 1.11 |
| CN | 0.66 | CONH₂ | 0.36 | 1.02 |
| CONH₂ | 0.36 | CONH₂ | 0.36 | 0.72 |
| CONH₂ | 0.36 | CN | 0.66 | 1.02 |
| CONH₂ | 0.36 | CO₂CH₃ | 0.50 | 0.86 |
| CN | 0.66 | CO₂C₆H₅ | 0.44 | 1.10 |
| CN | 0.66 | CF₃ | 0.54 | 1.20 |

Preferred examples of R⁹ include an aryl group, a heterocyclic group, and an alkyl group. Suitable among these groups is an aryl group, preferably a C₆₋₃₆, more preferably a C₆₋₃₀ aryl group. The aryl group is preferably an unsubstituted aryl group or an aryl group containing substituents selected from the group consisting of nitro group, a halogen atom, a cyano group, an acylamino group and a sulfonamido group.

Particularly preferred among these substituents is a chlorine atom.

Z preferably represents a hydrogen atom, a halogen atom, a C₆₋₃₆, preferably a C₆₋₂₄ arylthio group, a C₁₋₃₆, preferably a C₁₋₂₄ arylsulfinyl group, a C₁₋₃₆, preferably a C₁₋₂₄ heterocyclic thio group, or a nitroso group.

Preferred examples of Z include a hydrogen atom, a halogen atom (e.g., fluorine atom, chlorine atom, bromine atom), an unsubstituted arylthio group or an arylthio group containing substituents such as a halogen atom, an alkyl group, an alkoxy group, a cyano group, an aryloxycarbonyl group, an alkoxycarbonyl group, an ureido group, an alkoxycarbonylamino group, an acylamino group and a carboxyl group (e.g., 2-(n-butoxy)-5-(t-octyloxy)phenylthio, 2-(t-amylcarbonylamino)phenylthio, 2-(phenoxycarbonylamino)phenylthio, 2-(phenoxycarbonyl)phenylthio, 2-(3,3-dimethylureido)phenylthio, pentafluorophenylthio, pentachlorophenylthio,1,3,5-triisopropylphenylthio), an unsubstituted arylsulfinyl group or an arylsulfinyl group containing substituents such as a halogen atom, an alkyl group, an alkoxy group, a cyano group, an alkoxycarbonyl group and a carboxyl group (e.g., 2-(n-butoxy)-5-(t-octyloxy)phenylsulfinyl, 2-(t-amylcarbonylamino)phenylsulfinyl, 2-(phenoxycarbonylamino)phenylsulfinyl, 2-(phenoxycarbonyl)phenylsulfinyl, 2-(3,3-dimethylureido)phenylsulfinyl, pentafluorophenylsulfinyl, pentachlorophenylsulfinyl, 1,3,5-triisopropylphenylsulfinyl), a heterocyclic thio group or a nitroso group.

Preferred among these groups are a hydrogen atom, an arylthio group, and an arylsulfinyl group.

The compound represented by formula (III) also indicates various compounds in equilibrium as shown below:

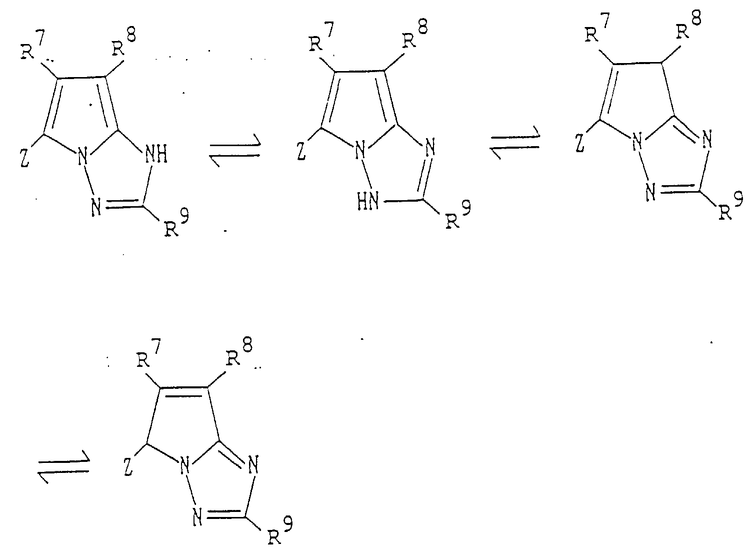

Specific examples of the compounds of the present invention will be set forth below, but the present invention should not be construed as being limited thereto.

Firstly, specific examples of the compounds represented by formulae (I) and (III) will be given. In the structural formulae shown below, (t)C₅H₁₁ indicates -C(CH₃)₂C₂H₅, and (t)C₈H₁₇ indicates -C(CH₃)₂CH₂C(CH₃)₃. M⁺ indicates the value (M⁺) of the master peak of mass spectrum. λmax(nm) indicates the maximum absorption wavelength of an ethyl acetate solution of a compound represented by formula (IV) when derived from the compound represented by formula (I):

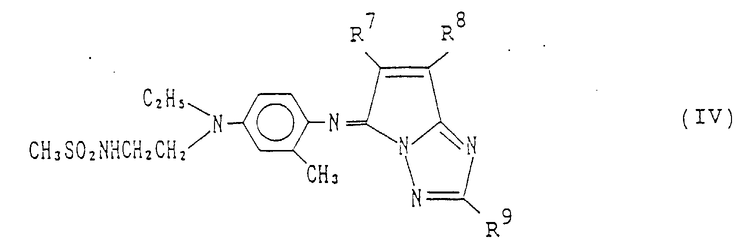

Some examples of the compounds represented by formula (III) will be shown with their melting points (m.p.).

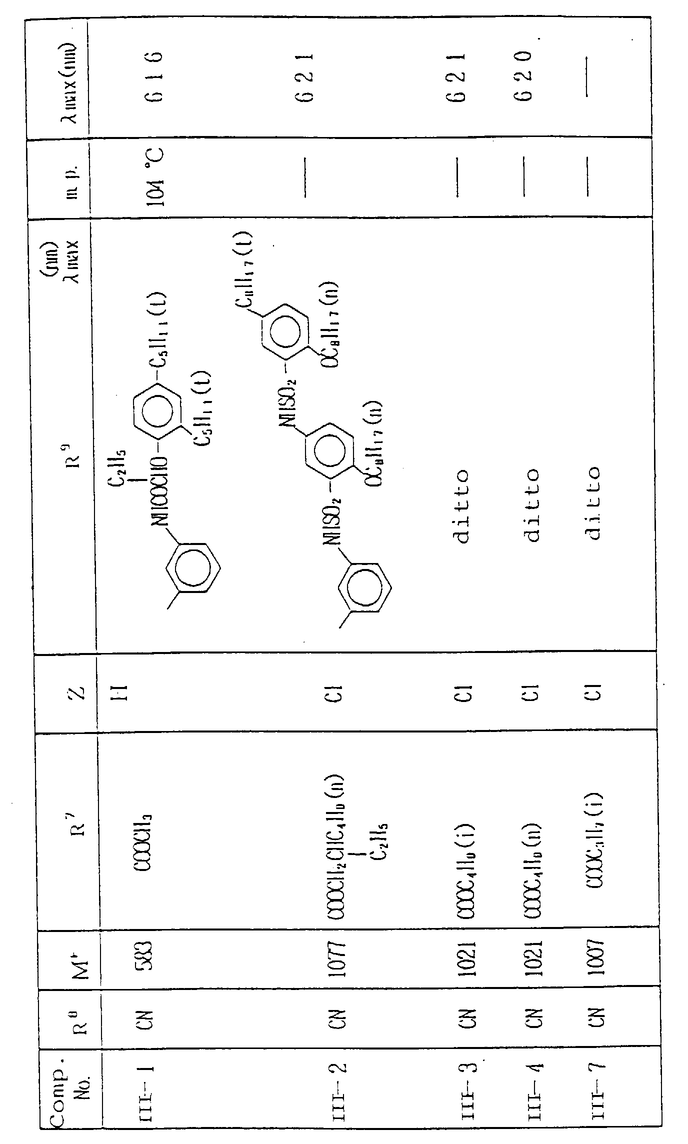

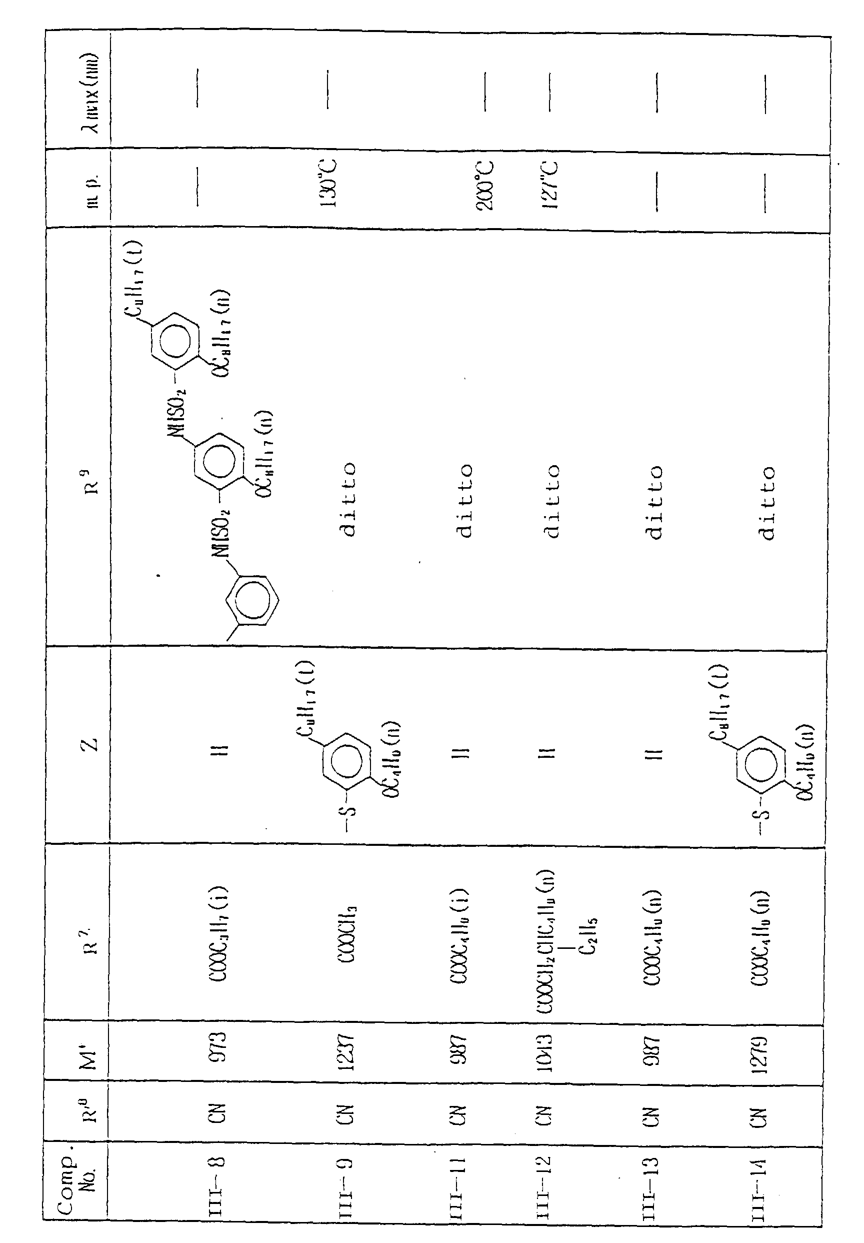

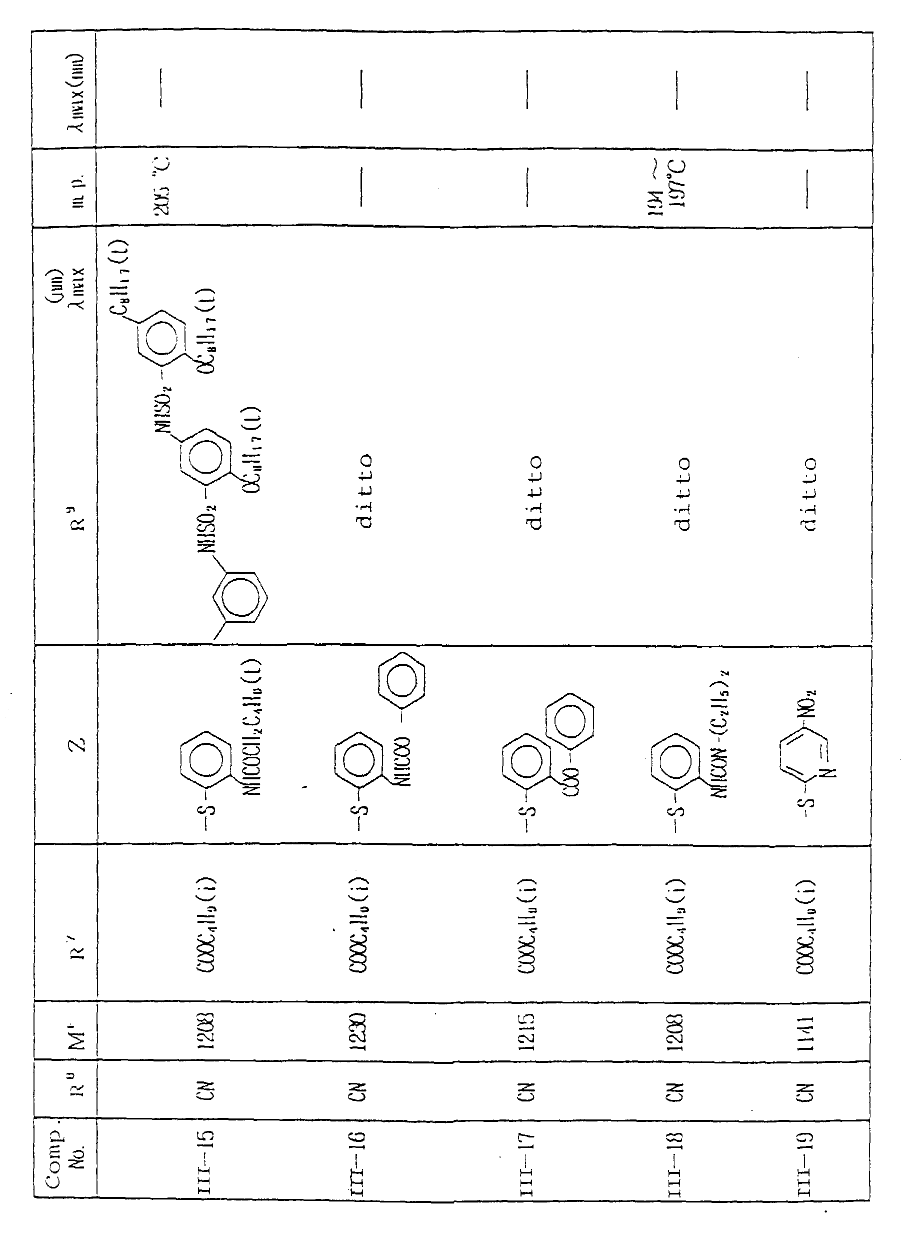

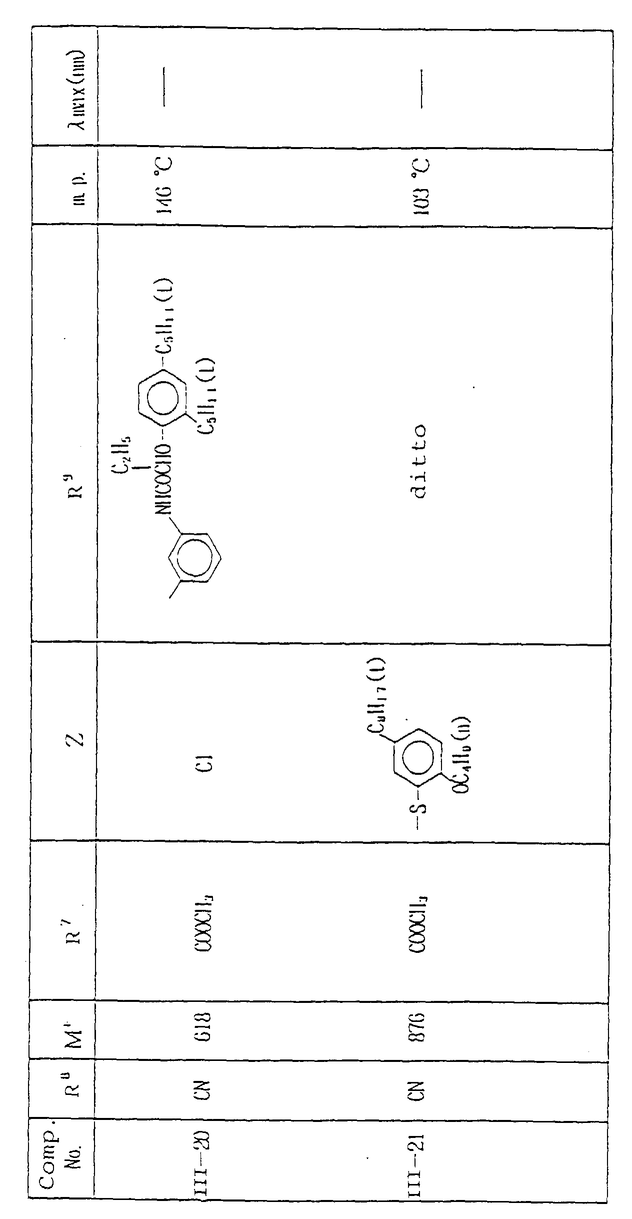

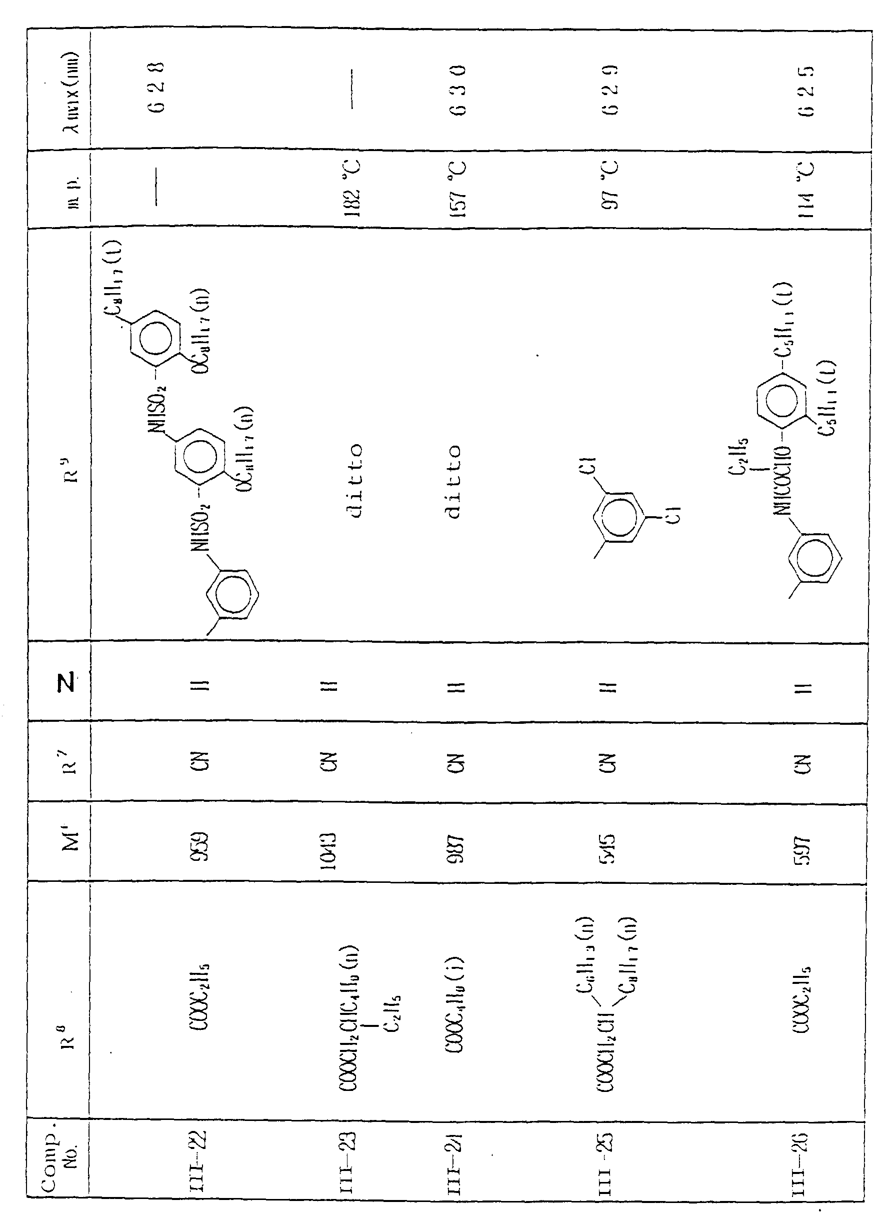

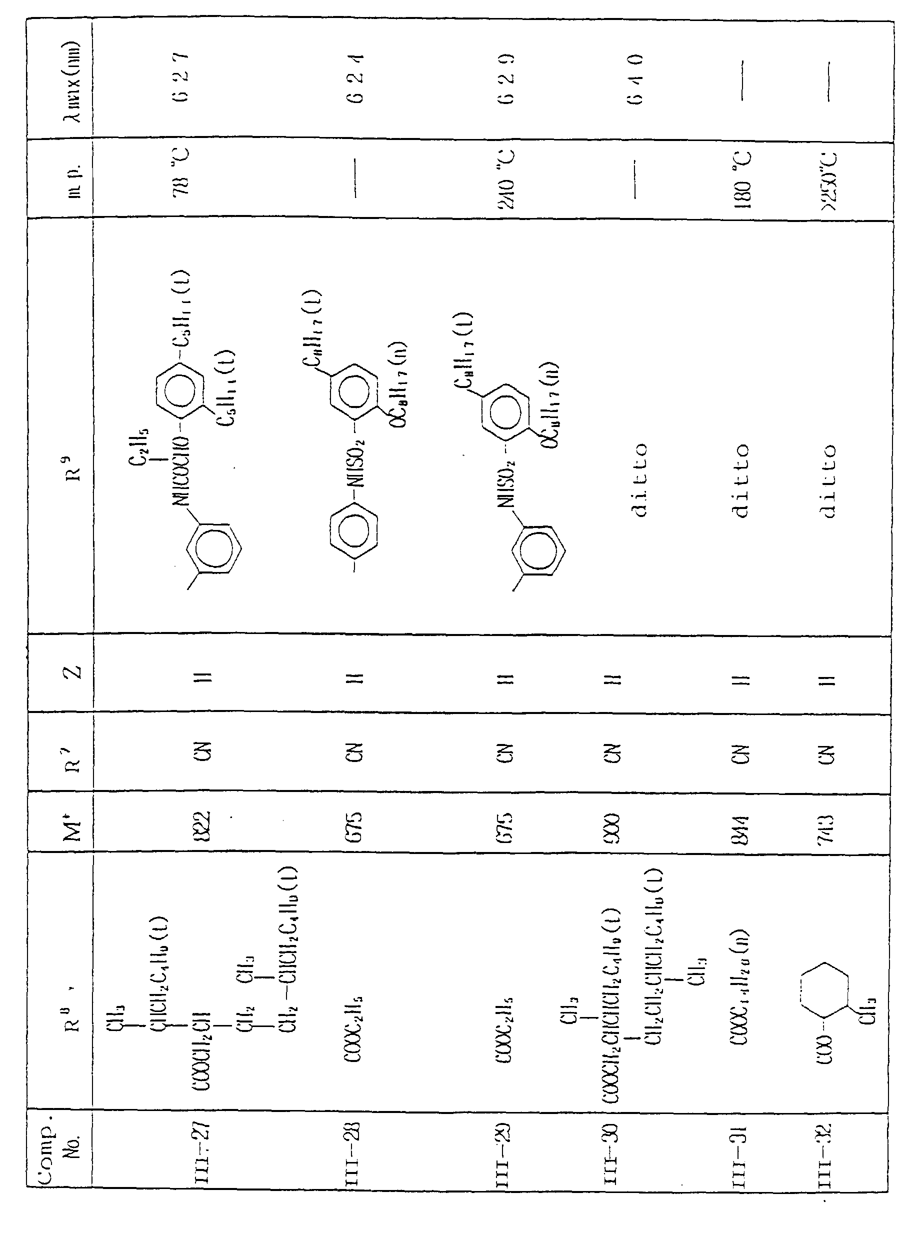

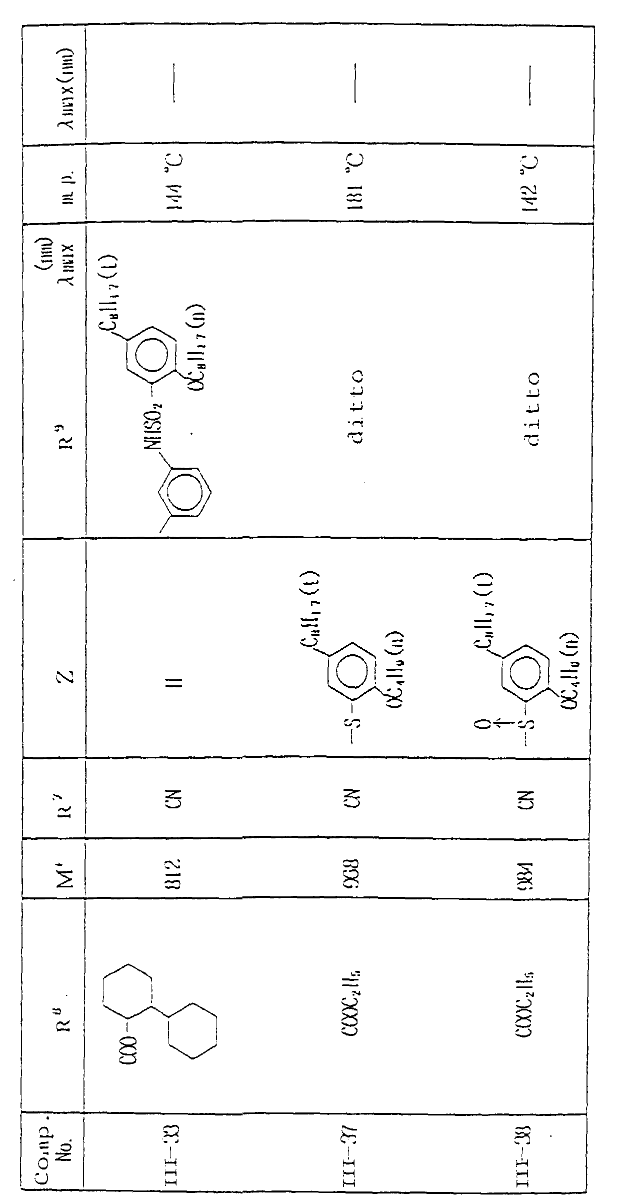

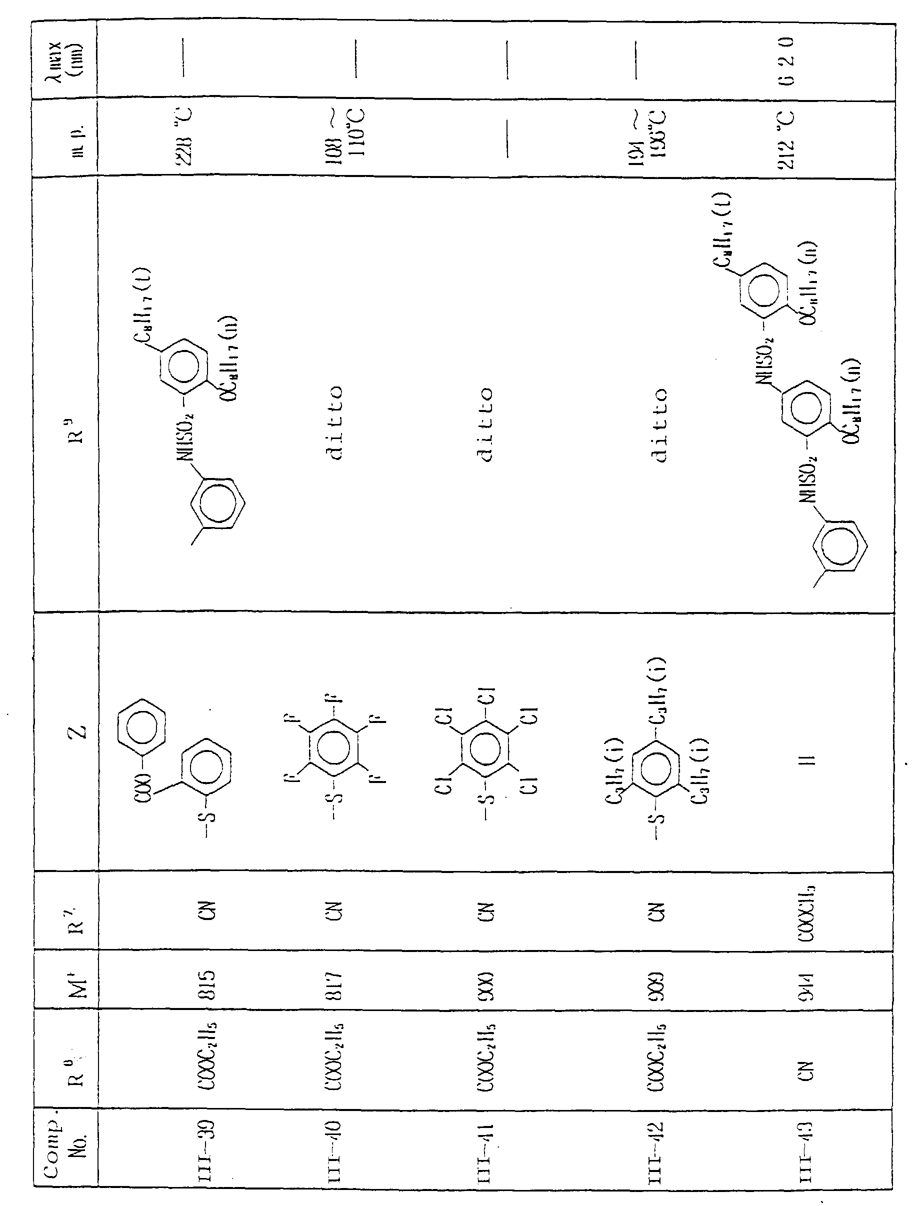

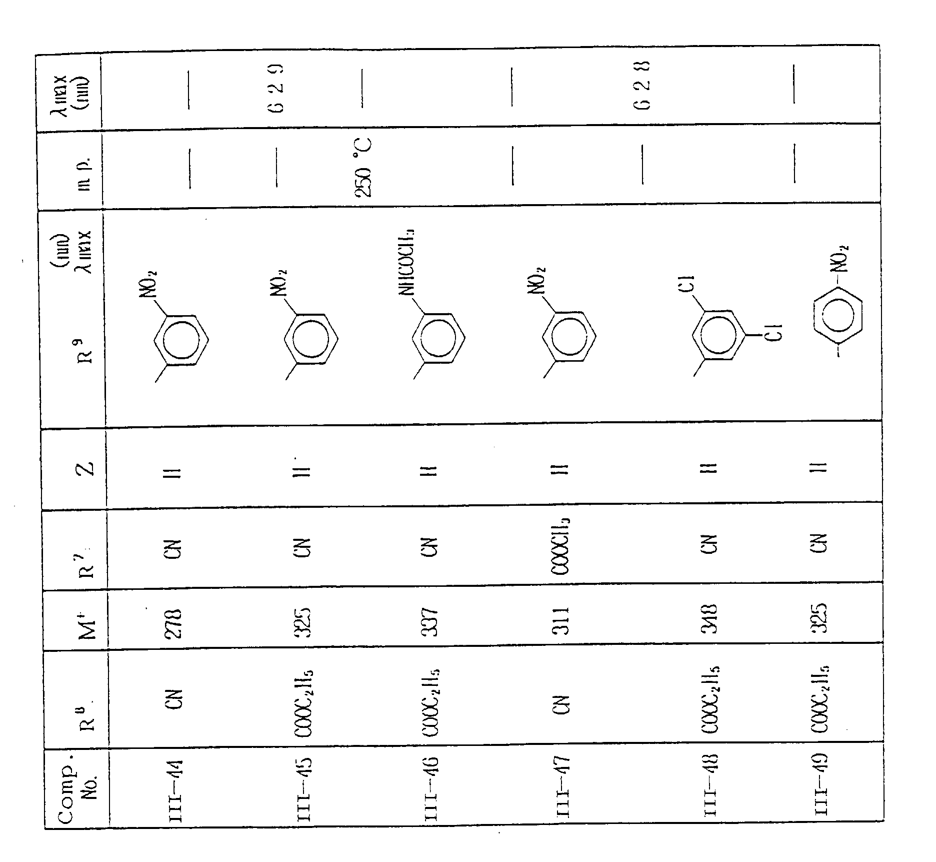

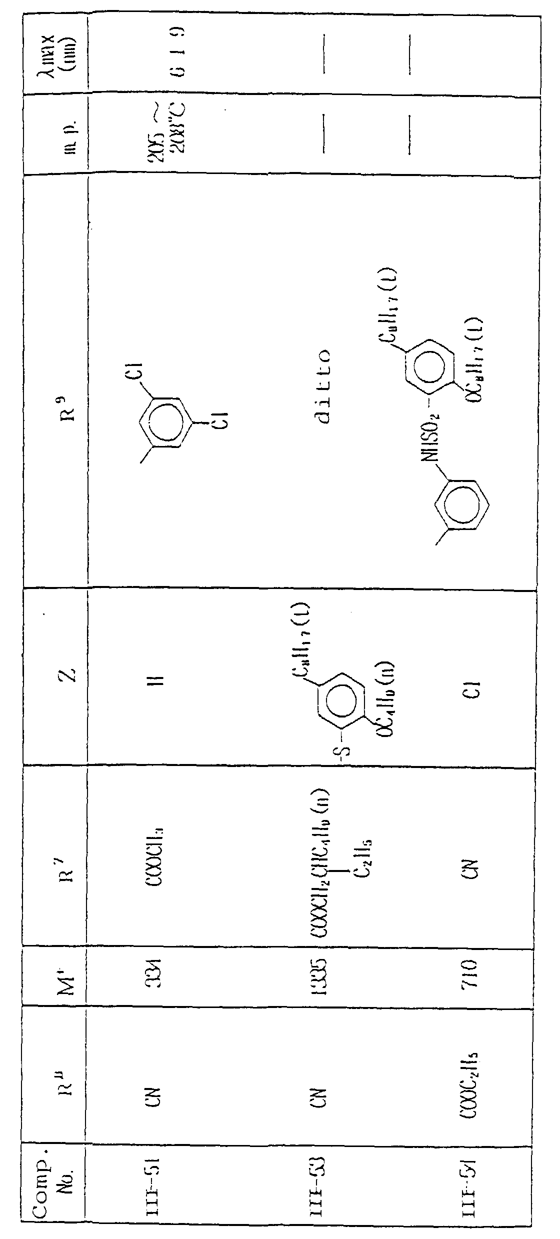

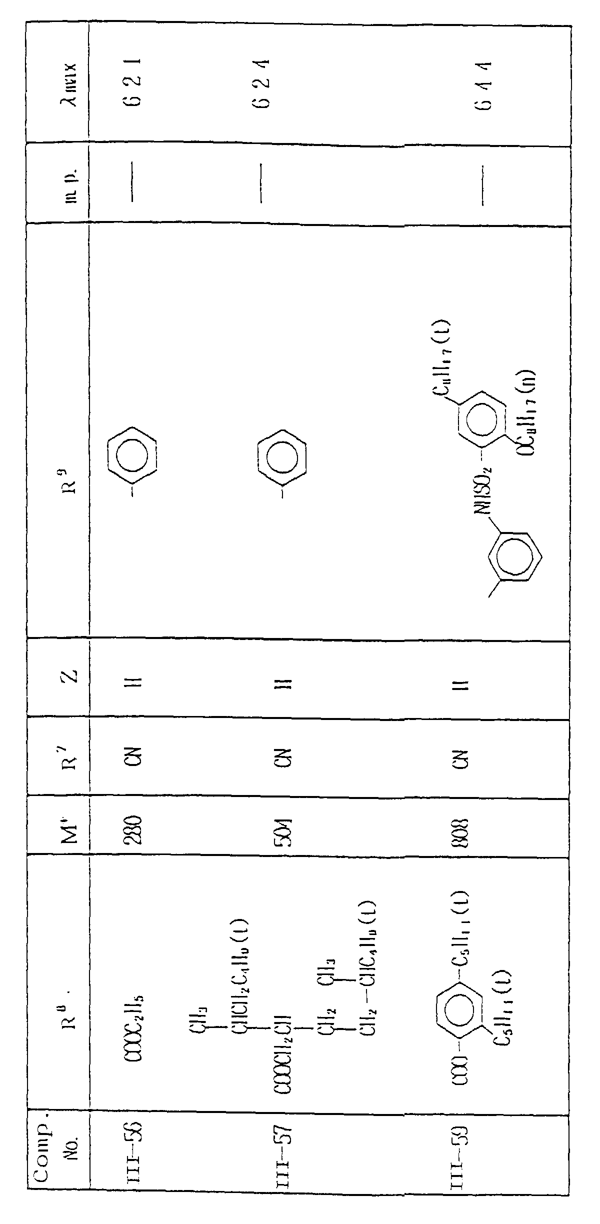

Specific examples of the compounds represented by formulae (I) and (II) will be given below. In the structural formulae shown below, Ph represents a phenyl group.

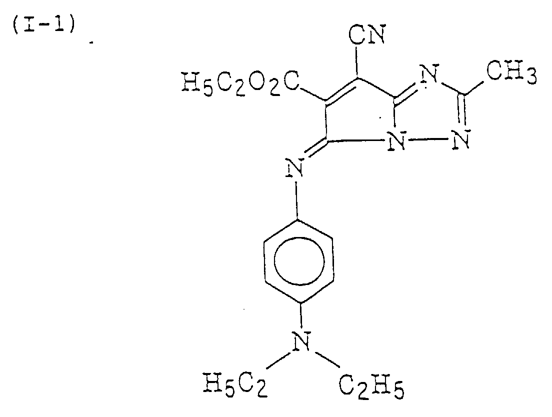

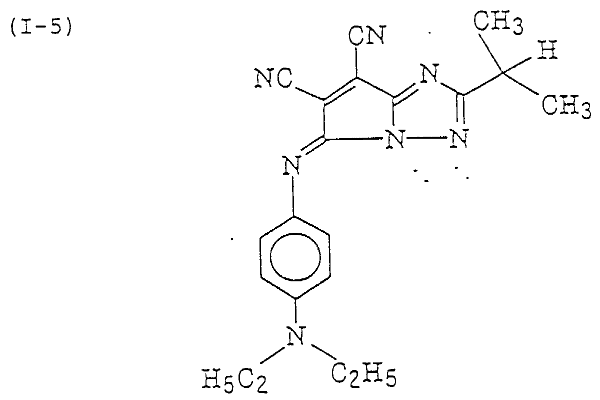

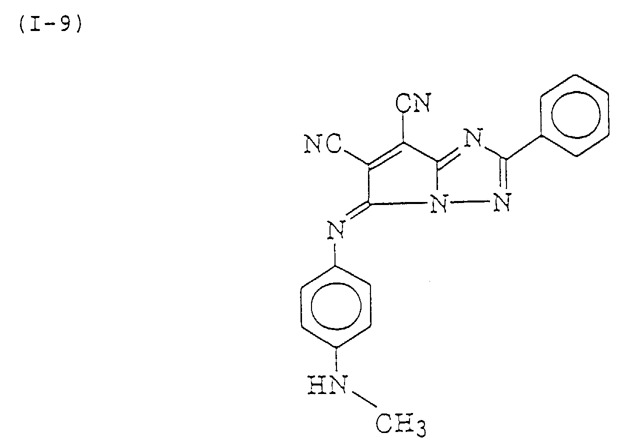

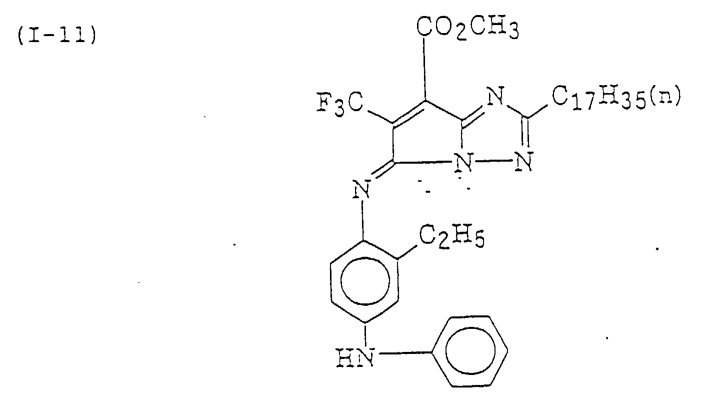

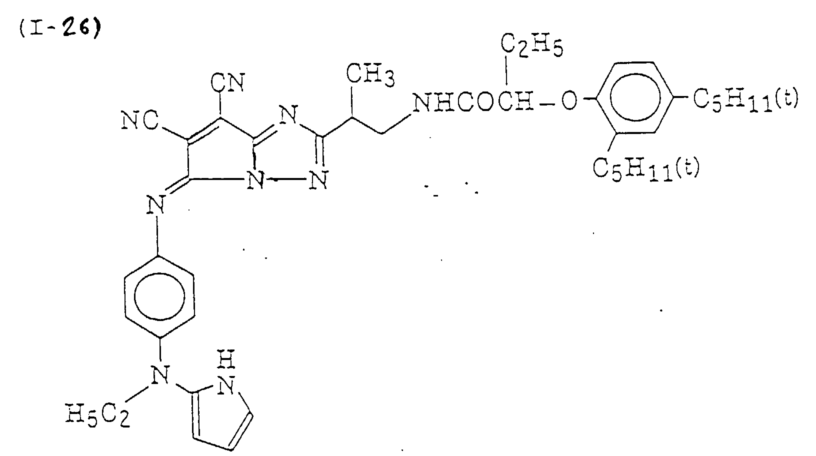

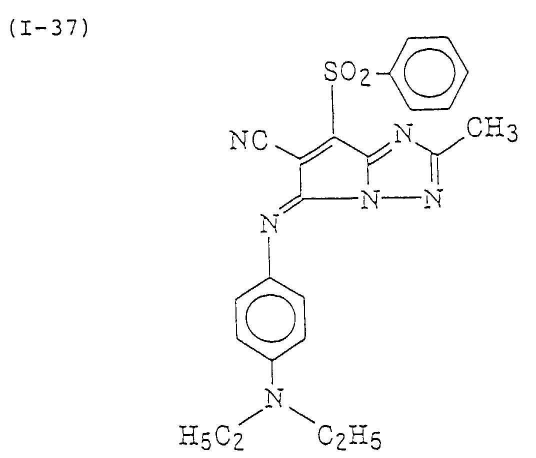

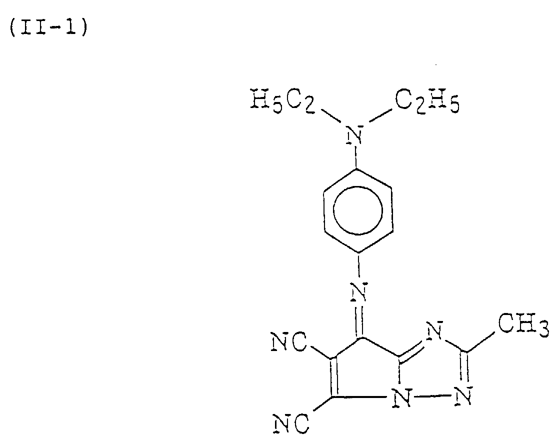

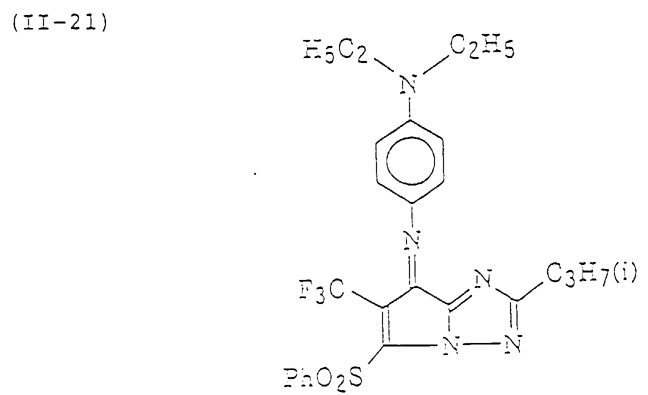

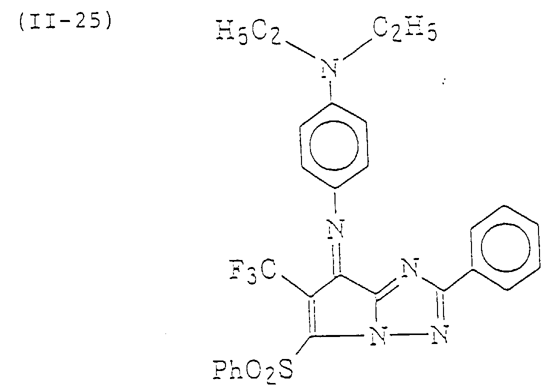

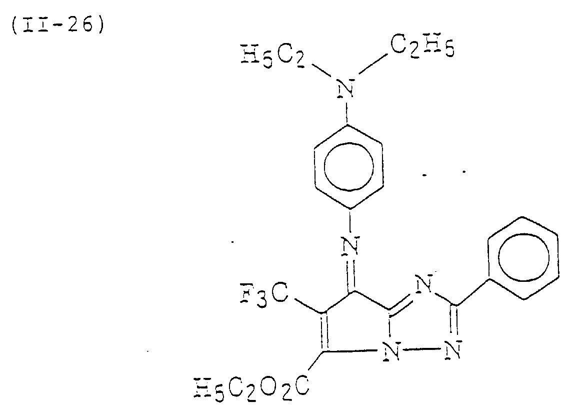

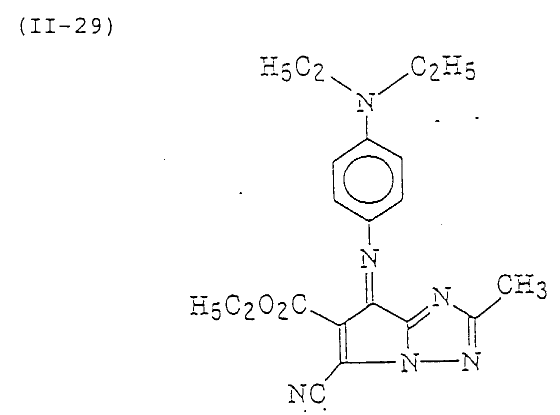

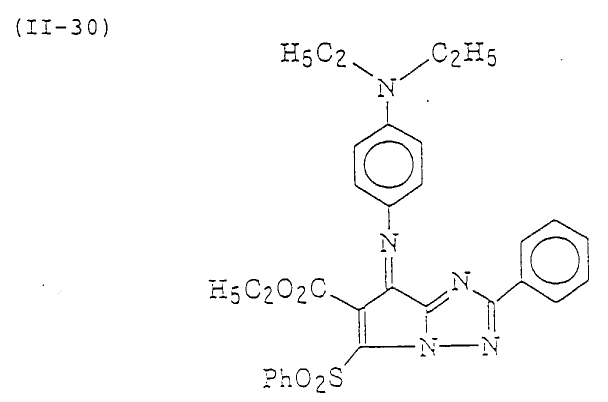

Typical examples of processes for the synthesis of compounds represented by formula (III) will be given below.
When R⁷ = COR: 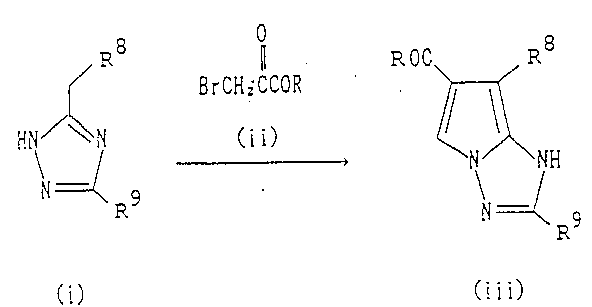 When R⁷ = CN:

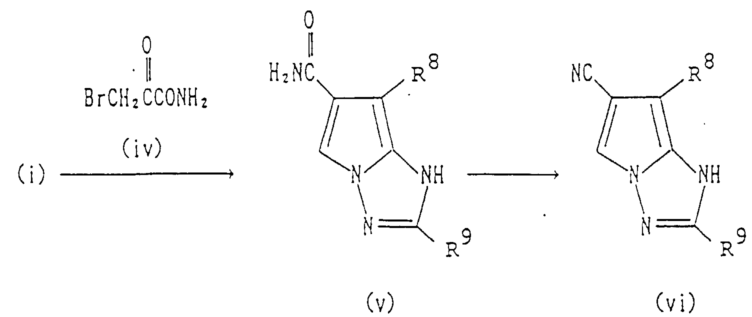

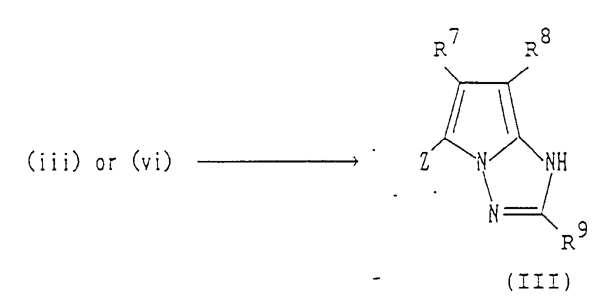

Compound (i) can be synthesized by any known method. For example, synthesis methods as described in J. C. S., 518 (1961), J. C. S., 5149 (1962), Angew. Chem., 72,956 (1960), and Berichte., 97,3436 (1964), and references cited therein, and analogous methods can be used.

The synthesis of Compound (iii) can be accomplished by the nucleophilic displacement reaction from Compound (i) to Compound (ii) and the subsequent cyclization reaction.

In this synthesis, a base such as sodium hydride, triethylamine, diazabicycloundecene, potassium carbonate and sodium carbonate may be preferably used. The reaction may be effected free of solvent or in a solvent such as tetrahydrofuran, acetonitrile, N,N-dimethylformamide, N,N-dimethylacetamide, N,N'-dimethylimidazolidon-2-one, acetone and toluene. The reaction temperature is normally in the range of -20°C to 150°C, preferably 0°C to 100°C.

The synthesis of Compound (v) can be accomplished by the nucleophilic displacement reaction from Compound (i) to Compound (iv) and the subsequent cyclization reaction as in the synthesis of Compound (iii).

Compound (vi) is derived by the dehydration reaction of Compound (v). In this reaction, phosphorus oxychloride, phosphorus pentachloride, phosphorus trichloride, etc. may preferably be used. The reaction may be effected free of solvent or in toluene or chloroform. The reaction temperature is normally in the range of - 10°C to 250°C, preferably 20°C to 200°C.

The compound represented by formula (III) can be synthesized from Compound (iii) or (vi) by a known method.

For example, if an arylthio group or heterocyclic thio group is connected to Compound (III) to obtain a 7-aromatic mercapto or heterocyclic mercapto substituted compound, a method described in U.S. Patent 3,227,554 can be used, i.e., a process can be used which comprises dissolving an arylmercaptan, heterocyclic mercaptan and their disulfides in a halogenated hydrocarbon solvent, treating the solution with chlorine or sulfuryl chloride to produce sulfenyl chloride, and then adding the solution to a solution of Compound (III) in an aprotonic polar solvent.

The compounds represented by formulae (I) and (II) can be synthesized by oxidation coupling of the following Compound A or B with the following Compound C:

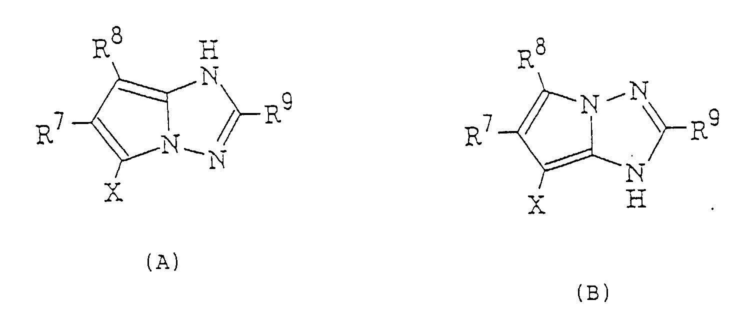

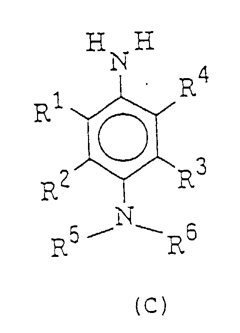

In these formulae, X represents a hydrogen atom or a split-off group which splits off upon coupling reaction, and formula (A) includes formula (III). It is clear that Compounds A and B may be in the form of their tautomers.

Alternatively, the compounds represented by formulae (I) and (II) can be synthesized by the dehydration condensation of the above mentioned Compound A or B with the following Compound D:

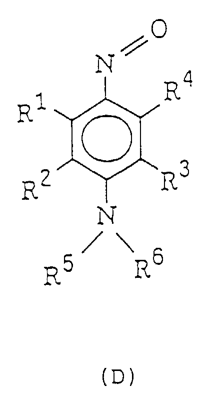

In this case, X in Compounds A and B is a hydrogen atom.

For example, the compound represented by formula (I) can be derived from the compound represented by formula (III) by a known method as described in JP-A-63-145281. That is, the compound represented by formula (I) can be synthesized in the presence of a compound represented by formula (III), a phenylenediamine, and an oxidizer.

It is thought that this coupling reaction involves a nucleophilic attack of a coupler anion on the quinodimine produced by the oxidation of phenylenediamine to form a leuco dye which is then converted to an azomethine dye as described in T. H. James, The Theory of the Photographic Process, 4th ed., Macmillan, 1977. The reaction preferably proceeds under basic condition. The reaction medium may be any of an organic medium, an aqueous organic medium and an aqueous solution. If the reaction is effected in a basic aqueous solution, the compound represented by formula (III) may be an oil-in-water dispersion. The oil-in-water dispersion may be present in a hydrophilic colloidal medium such as gelatin. As the oxidizer there may be used any organic or inorganic oxidizer having a potential strong enough to oxidize phenylenediamine. The oxidizer may be used in the form of a solution or a dispersion in the reaction medium. If Z in formula (III) is a hydrogen atom, phenylenediamine is present in the system in an amount of 0.1 to 10 mol, preferably 0.5 to 2 mol per mol of compound of formula (III), and the oxidizer is used in an amount of at least 4 equivalents, preferably 4.4 to 20 equivalents. If Z is not a hydrogen atom, the compound represented by formula (I) can be synthesized in the same manner as above except that the oxidizer is used in an amount of at least 2 equivalents, preferably 2.2 to 10 equivalents. If the reaction medium is aqueous, coupling may be effected at a pH of 8 or more, preferably 10 to 12. As the oxidizer there can be used silver halide, hydrogen peroxide, manganese dioxide, potassium persulfate, oxygen, or compounds as described in Fieser & Fieser, Organic Reagents.

The present invention will be further described in the following examples, but the present invention should not be construed as being limited thereto.

### EXAMPLE 1: Synthesis of Compound (III-1)

Compound (III-1) was synthesized in the following process:

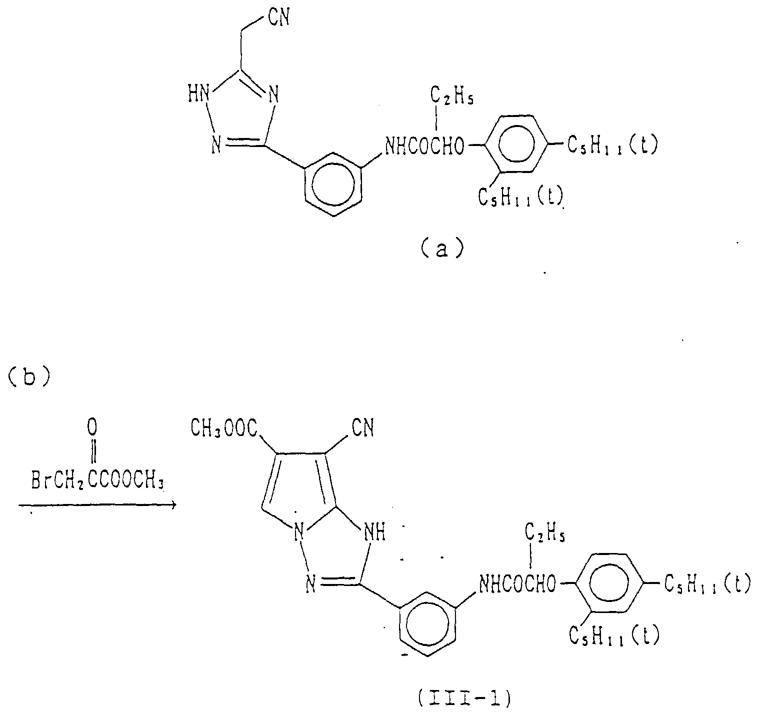

To a solution of Compound (a) (50.1 g; 0.1 mol) and Compound (b) (19.9 g; 0.11 mol) in tetrahydrofuran (300 ml) was added gradually sodium hydride (12.0 g; 0.30 mol) at a temperature of 0°C in such a manner that the reaction temperature didn't rise. The reaction system was stirred for 1 hour. 200 ml of a 1 N dilute hydrochloric acid was added to the reaction system. The reaction system was extracted with 300 ml of ethyl acetate three times. The resulting organic phase was washed with water and saturated brine, and then dried over anhydrous sodium sulfate. The solvent was distilled off under reduced pressure. The material was purified through column chromatography (developing solvent: 3 : 1 mixture of hexane and ethyl acetate) to obtain 7.57 g of Compound (III-1) (yield: 13%).

### EXAMPLE 2: Synthesis of Compound (III-22)

Compound (III-22) was synthesized in the following process:

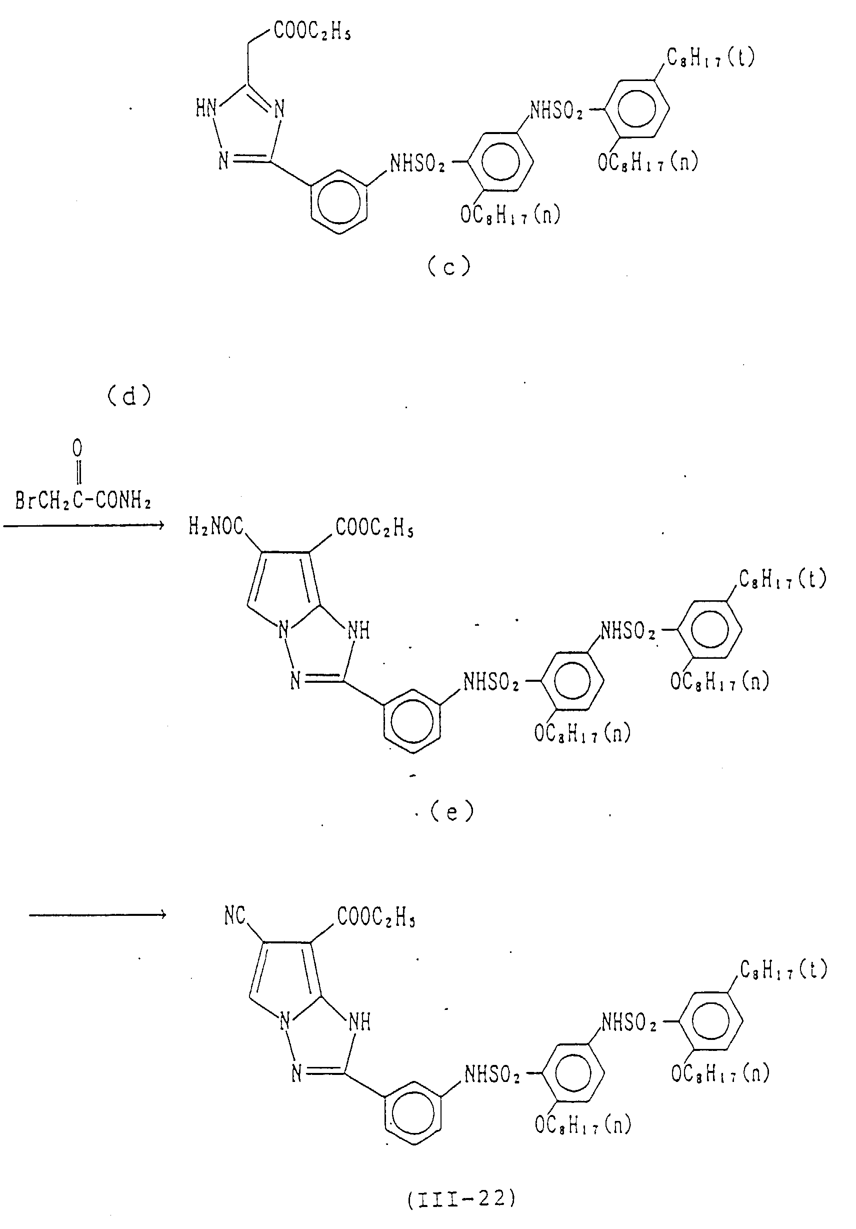

From Compound (c) (45.5 g; 50 mmol) and Compound (d) (9.13 g; 55 mmol) Compound (e) was obtained in the same manner as in Synthesis Example 1 (3.90 g; 8%). To Compound (e) (3.90 g; 4.0 mmol) was added phosphorus oxychloride (1.47 g; 9.6 mmol) at a temperature of 100°C. The reaction system was stirred for 1 hour. After the reaction system was cooled, 10 cc of water was added thereto. The reaction system was extracted with 30 cc of ethyl acetate three times. The resulting organic phase was washed with saturated brine, and then dried over anhydrous sodium sulfate. The solvent was distilled off under reduced pressure. The material was recrystallized from acetonirile (30 cc) to obtain 3.22 g of Compound (III-22) (yield: 84%).

### EXAMPLE 3: Synthesis of Compound (III-20)

Compound (III-20) was synthesized in the following process:

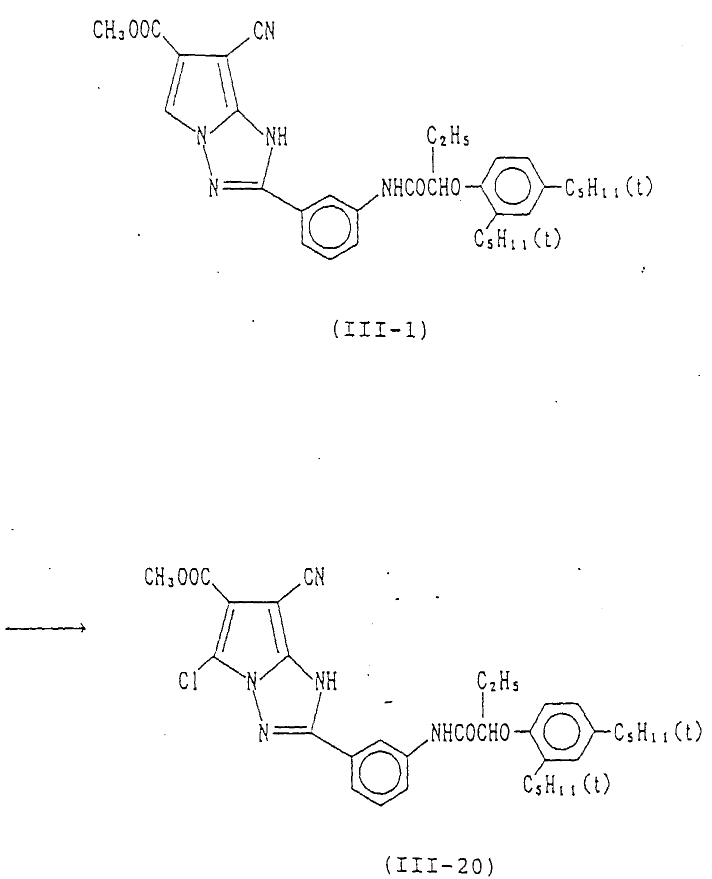

To a solution of Compound (III-1) (2.91 g; 5 mmol) in methylene chloride (30 ml) was added dropwise sulfuryl chloride (0.74 g; 5.5 mmol) at a temperature of 0°C. The reaction system was stirred for 1 hour. Water was added to the reaction system. The reaction system was extracted with 30 cc of methylene chloride three times. The resulting organic phase was washed once with saturated brine, and then dried over anhydrous sodium sulfate. The solvent was distilled off under reduced pressure. The material was recrystallized from acetonirile to obtain 2.83 g of Compound (III-20) (yield: 92%).

### EXAMPLE 4: Synthesis of Compound (III-21)

Compound (III-21) was synthesized in the following process:

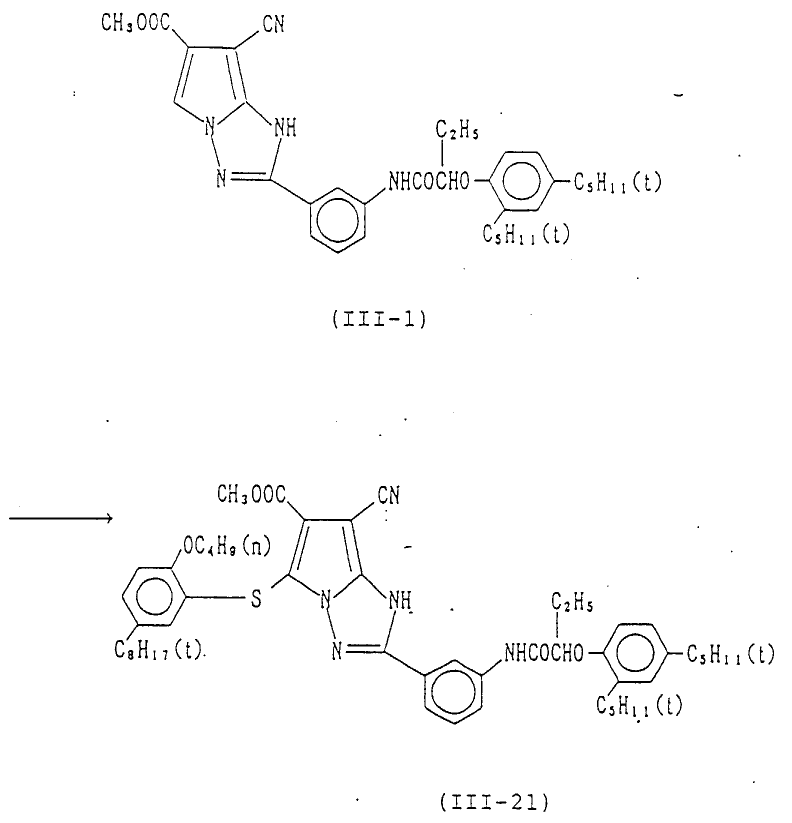

To a solution of di-[(2-n-butoxy-5-t-octyl)phenyl]disulfide (3.53 g; 6 mmol) in methylene chloride (20 ml) was added sulfuryl chloride (1.76 g; 13 mmol) at a temperature of 0°C. The reaction system was stirred for 1 hour. Methylene chloride and sulfuryl chloride were removed under reduced pressure. To the residue was added methylene chloride (20 ml). The solution was added dropwise to a solution of Compound (III-1) (2.91 g; 5 mmol) in N,N-dimethylformamide (10 ml) at a temperature of 0°C. The reaction system was stirred for 1 hour. Water was added to the reaction system. The reaction system was extracted with 30 ml of ethyl acetate twice. The resulting organic phase was washed with water and saturated brine, and then dried over anhydrous sodium sulfate. The solvent was distilled off under reduced pressure. The material was purified through silica gel chromatography (developing solvent: 2 : 1 mixture of hexane and ethyl acetate) to obtain 2.93 g of Compound (III-21) (yield: 67%).

### EXAMPLE 5: Synthesis of Compound (III-25)

Compound (III-25) was synthesized in the following process:

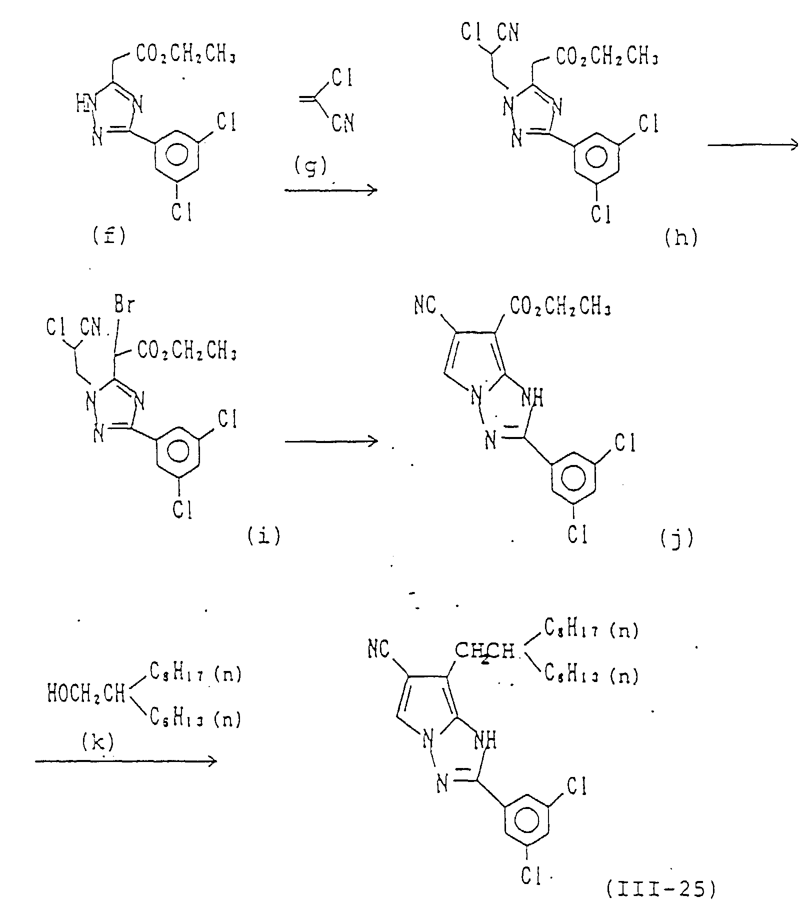

30.0 g of Compound (f) and 11.4 g of Compound (g) were dissolved in 300 ml of tetrahydrofuran. 6.0 ml of tetramethyl guanidine was added to the solution. The material was stirred at room temperature for 5 hours. 500 ml of ethyl acetate was added to the material. The material was then washed with water. The ethyl acetate was dried, and then distilled off. The material was then crystallized from methanol to obtain 27.5 g of Compound (h).

27.4 g of Compound (h) thus obtained was dissolved in 270 ml of tetrahydrofuran. 13.6 g of bromine was gradually dropwise added to the solution under cooling with ice. The material was stirred for 5 hours. 500 ml of ethyl acetate was added to the material. The material was then washed with water until it turned neutral. The resulting ethyl acetate phase was dried, and then distilled off. The residue was purified through column chromatography to obtain 28.0 g of Compound (i).

28.0 g of Compound (i) thus obtained was dissolved in 170 ml of tetrahydrofuran. 9.6 g of 60% sodium hydride was gradually added to the material while the reaction temperature was kept at - 10°C. After the completion of the reaction, ethyl acetate was added to the reaction system. The material was then washed with water. The resulting ethyl acetate phase was dried, and then distilled off. The material was purified through column chromatography to obtain 3.0 g of Compound (j).

1.8 g of Compound (j) thus obtained and 12.4 g of Compound (k) were dissolved in 2.0 ml of sulfolane. To the solution was further added 1.5 g of titanium isopropoxide. The reaction system was then allowed to undergo reaction for 1.5 hours while the reaction temperature was kept at 110°C. Ethyl acetate was then added to the material. The material was then washed with water. The resulting ethyl acetate phase was dried, and then distilled off. The residue was then puridied through column chromatography to obtain 1.6 g of Compound (III-25).

The melting point of Compound (III-25) was 97 to 98°C.

### EXAMPLE 6: Synthesis of Dye of Compound (III-20)

To a solution of Compound (III-20) (9.59 g; 10.0 mmol) in 100 ml of a 1 : 1 mixture of ethyl acetate and ethanol was added 40 ml of an aqueous solution of sodium carbonate (9 g). Subsequently, 4-(N-ethyl-N-(2-methanesulfonamidoethyl)amino)-2-methylaniline sulfate (6.38 g; 12.2 mmol) was added to the system. The material was then stirred at room temperature for 5 hours. To the material was then added dropwise 20 ml of an aqueous solution of ammonium persulfate (4.5 g). The material was then stirred for 1 hour. The material was then extracted with ethyl acetate three times. The resulting organic phase was washed with saturated aqueous solution of sodium chloride, and then dried over anhydrous sodium sulfate. The solvent was then distilled off under reduced pressure. The material was then purified through column chromatography (developing solvent: 20 : 1 mixture of methylene chloride and methanol). The material was then recrystallized from methanol to obtain 10.2 g of a dye of Compound (III-20) (yield: 83%).

### EXAMPLE 7: Synthesis of Compound (I-2)

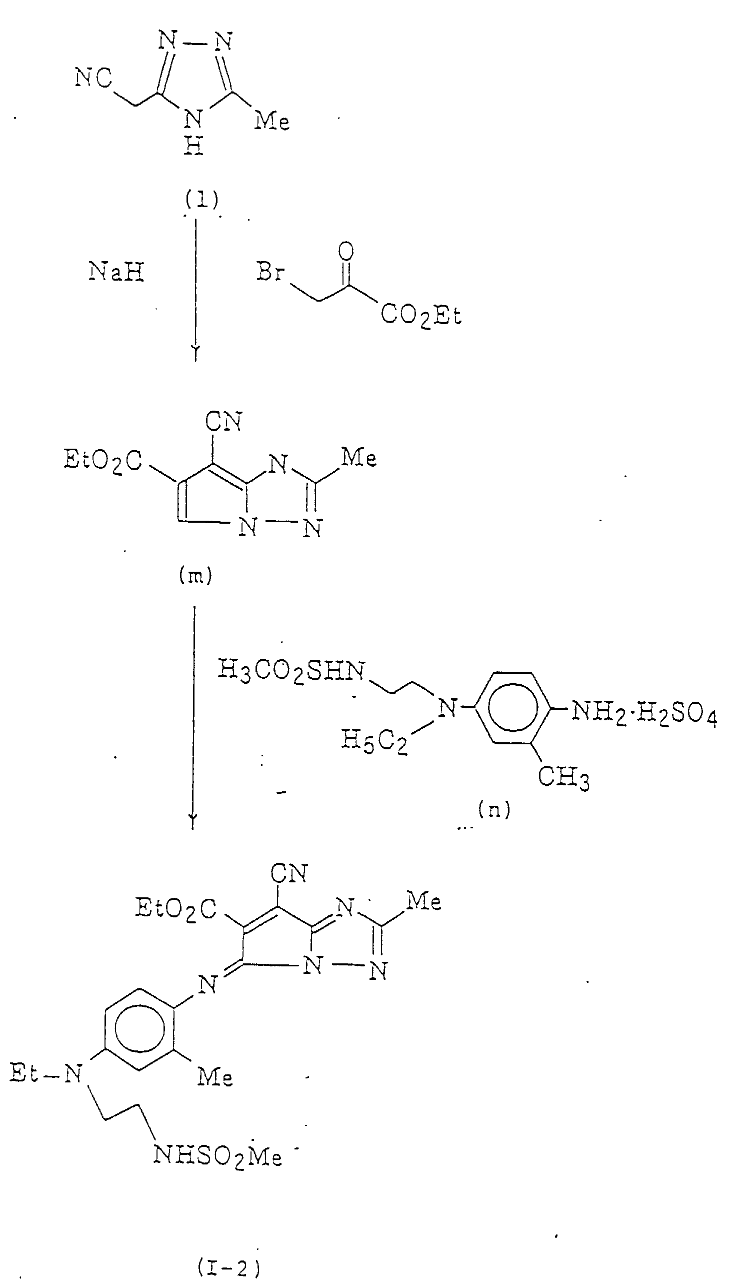

1.5 g of 3-cyanomethyl-5-methyl-1,2,4-triazole (Compound (ℓ)) was dissolved in 30 ml of tetrahydrofuran. To the solution was added 1.0 g (24.6 mmol) of sodium hydride (60% in oil). The material was then heated to a temperature of 50°C. To the material was added 1.9 ml (14.7 mmol) of ethyl bromopyruvate. The material was further heated under reflux for 2 hours. The material was then cooled to room temperature. To the material were then added brine and 2 N hydrochloric acid solution to acidify the solution. The material was extracted with ethyl acetate twice, and then dried. Ethyl acetate was then distilled off under reduced pressure. The residue was then purified through silica gel chromatography to obtain 1.20 g of Compound (m) (yield: 45%).

The synthesis of Compound (ℓ) was accomplished by a method described in Journal of the Chemical Society, 5149 (1962).

To 1.0 g of Compound (m), 20 cc of ethyl acetate, 20 cc of ethanol, 24 cc of water, 6.6 g of sodium carbonate and 3.1 g of Compound (n) was added a solution of 4.6 g of ammonium persulfate in 5 cc of water with stirring.

The reaction system was then allowed to undergo reaction at a temperature of 20°C for 1 hour. The material was then extracted with ethyl acetate. The resulting organic phase was washed with water twice, and then dried over magnesium sulfate. After filtration, the solvent was distilled off. The resulting crude crystal was purified through silica gel chromatography, and then recrystallized from methanol to obtain 0.82 g of Compound (I-2) (yield: 37%; m.p. 223 to 224°C)

The absorption characteristics of Compound (I-2) of the present invention in a solvent are shown in Fig. 1 together with those of Comparative Dye a.

In Fig. 1, the solid line indicates the absorption characteristics of Compound (I-2) of the present invention, and the broken line indicates the absorption characteristics of Comparative Dye a.

The maximum absorption wavelength (λmax) and half-value width of Compound (I-2) of the present invention and Comparative Dye a are set forth below.

### Compound (I-2) of the present invention

λmax: 602.3 nm (in ethyl acetate)
Half-value width: 94.9 nm

### Comparative Dye

λmax: 562.6 nm (in ethyl acetate)
Half-value width: 88.1 nm

As the comparative dye there was used Comparative Dye a shown below:

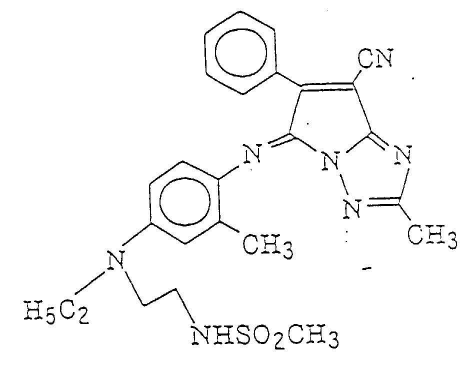

Fig. 1 shows that the dye of the present invention exhibits a sharp absorption, a reduced undesirable absorption in the yellow portion and a maximum absorption suitable as cyan dye.

On the other hand, it is shown that Comparative Dye a wherein the substituent R⁷ in the coupler portion is a phenyl group (σ_{P} is smaller than 0.15) doesn't exhibit cyan color.

### REFERENTIAL EXAMPLE 1

The dyes of the present invention were examined for fastness to light in a solution system. The dyes thus examined and the test results are set forth in Table 1.
- Condition:: merry-go-round type xenon radiator (produced by Dojun Koki K.K.; output: 500 W, 100,000 lux)
- Cell:: quartz cell
- Solvent:: acetonitrile
- Concentration:: 3.0 x 10⁻⁵ (mol/ℓ)
- Percent residue:: represented by the change (%) in the concentration at λmax from before to after forced discoloration test

**Table 1**

| Dye | Irradiation Time | Ultraviolet Light Filter | Percent Residue | Remarks |
|---|---|---|---|---|
| b | 60 min. | None | 43 | Comparison |

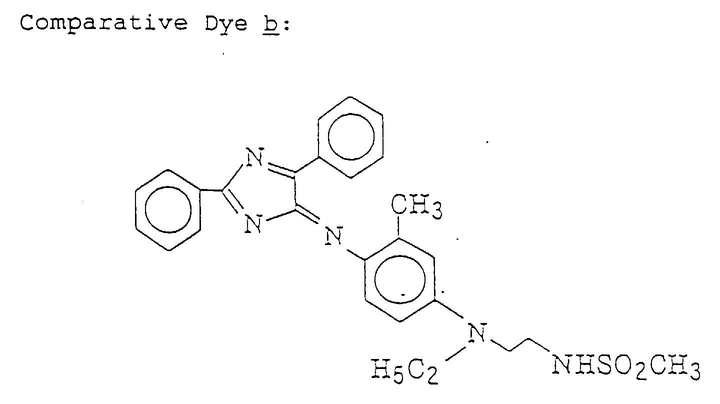

### REFERENTIAL EXAMPLE 2

In order to demonstrate the usefulness of the dyes of the present invention as filter dyes, the following model filters were prepared and examined for fastness to light.

Onto a 100-µm thick polyethylene terephthalate film (produced by Teijin Limited) which had been rendered heat resistant and smoothened on its back side as a support was coated a dye dispersion layer coating composition having the following formulations by a wire bar coating process to prepare a model filter. The thickness of the coating was adjusted such that the density after drying reached 2.0.

| Dye dispersion layer coating composition: | |
|---|---|
| Dye (I-1) | 1.0 g |
| Polyvinyl butyral resin (Denka Butyral 5000-A produced by Denki Kagaku Kogyo K.K.) | 3.0 g |
| Toluene | 50 cc |
| Methyl ethyl ketone | 50 cc |
| Polyisocyanate (Takenate D110N produced by Takeda Chemical Industries, Ltd.) | 0.2 cc |

Model filters were prepared in the same manner as above except that Dye (I-1) was replaced by other dyes set forth in Table 2.

The model filters thus prepared were irradiated with light from a 17,000 lux fluorescent lamp for 14 days to examine the stability of the dyes. For evaluation of the dye stability, the ratio of the density measured before to after irradiation was determined. The results are set forth in Table 2.

**Table 2**

| Dye | Percent REsidue | Remarks |
|---|---|---|
| I-1 | 95 | Invention |
| b | 85 | Comparison |

The results set forth above show that the dyes of the present invention exhibit a remarkable fastness to light as compared with the comparative dyes.

### REFERENTIAL EXAMPLE 3

Onto a commercially available uncoated base paper (basis weight: 64 g/m²) was coated a coating solution consisting of 43 parts (by weight calculated in terms of solid content, hereinafter the same) of finely divided hollow grains of a styrene-acrylic ester copolymer (grain diameter: 0.3 to 0.4 µm), 17 parts of vapor phase process anhydrous silica (grain diameter: 12 nm), 12 parts of a styrene-butadiene copolymer latex, 18 parts of a polyvinyl acetate latex and 10 parts of finely divided grains of polymethyl methacrylate (grain diameter: about 8 µm) by means of a wire bar in such an amount that the solids content reached 10 g/m² to prepare a paper for ink jet recording.

Ink jet recording was made on this recording paper with Ink A having the following formulations. In this ink jet recording, an electrostatic acceleration type ink jet apparatus equipped with a head having a nozzle pore diameter of 50 µm and a dot density of 8/mm was used.

| Ink A: | |
|---|---|
| Dye (I-1) of the present invention | 6 g |
| Diethyl phthalate | 30 g |
| Diisopropyl adipate | 44 g |
| N,N-Diethyldodecanamide | 20 g |

This ink exhibited an excellent dischargeability, and a sharp and high density cyan image was obtained.

After these images were allowed to stand under indoor light for 3 months, the density thereof showed a drop of 1% or less.

### REFERENTIAL EXAMPLE 4

### (Preparation of heat transfer material)

The following materials were sufficiently dispersed in admixture to prepare a coating solution for smooth heat-resistant protective layer.

| Formulations of coating solution: | |
|---|---|
| Methyl methacrylate | 10 g |
| n-Butyl acrylate | 2 g |
| Benzoyl peroxide | 0.1 g |
| Silica | 35 g |
| Isopropyl alcohol (IPA) | 15 g |

The coating solution was diluted with a proper amount of a mixture of toluene and IPA. The coating solution was then coated on a 6-µm thick polyethylene terepthalate film (hereinafter referred to as "PET") as a substrate by means of a wire bar. The coated material was then dried at a temperature of 100°C for 1 minute to form a smooth heat-resistant protective layer with a thickness of about 1.5 µm.

A hot-melt ink having the following formulation was prepared.

| Formulation of hot-melt ink: | |
|---|---|
| Dye (I-1) | 10 g |
| Barium salt of lanolin fatty acid | 30 g |
| Carnauba wax | 20 g |
| Paraffin wax | 20 g |
| Dispersant | 0.5 g |
| Liquid paraffin | 5 g |

The ink having the above mentioned formulation was then sufficiently dispersed with a mixture of 100 cc of methyl ethyl ketone and 130 cc of toluene at a temperature of 68°C by a ball mill for about 48 hours.

To the ink dispersion was added 300 g of a 20 wt.% vinyl chloride-vinyl acetate copolymer resin solution (10 parts of resin, 20 parts of toluene, 20 parts of methyl ethyl ketone). The mixture was then dispersed by a ball mill for about 1 hour to prepare a coating solution of heat-sensitive transfer composition.

This coating solution was coated on the surface of the above mentioned PET film opposite the smooth heat-resistant protective layer by means of a wire bar, and then dried at a temperature of 100°C for 1 minute to form a hot-melt ink layer having a thickness of about 5 µm.

The heat transfer material thus obtained was laminated with a plain paper as a heat transfer image-receiving material in such an arrangement that the transfer layer (hot-melt ink layer) in the heat transfer material was brought into contact with the plain paper. Printing was effected by a heat head on the heat transfer material from the support side to cause transfer. As a result, a sharp cyan color recording was provided.

These recorded sheets were then examined for stability of image to light. A high image stability to light was obtained.

The derivatives represented by formula (III) of the present invention are useful as synthesis intermediates of physiologically active substances such as pharmaceutical preparations and pesticides. These derivatives are also useful as couplers in the field of photographic chemistry.

On the other hand, the compounds represented by formulae (I) and (II) are useful dyes which are advantageous in that they exhibit a reduced secondary absorption and a primary absorption wavelength of 600 nm or more.

When used as an image forming dye in ink jet recording process, heat-sensitive transfer recording process, etc., the novel pyrrolotriazole azomethine dye of the present invention provides a high density image free of discoloration because of its remarkably excellent fastness to light.

Also, when used as various filter dyes, the azomethine dye of the present invention provides a filter with an excellent stability and a remarkably reduced density drop because of its excellent fastness to light.

Furthermore, when image formation is effected on a heat transfer dye providing material comprising the pyrrolotriazole azomethine dye of the present invention, it provides an image with a high fastness to heat and light and an excellent color reproducibility.

Moreover, the pyrrolotriazole azomethine dye of the present invention provides the heat transfer dye providing material with a better storage stability when used in admixture with one or more present azomethine dyes than when used singly.

## Claims

1. A pyrrolotriazole derivative represented by any one of formulae (I), (II), or (III): 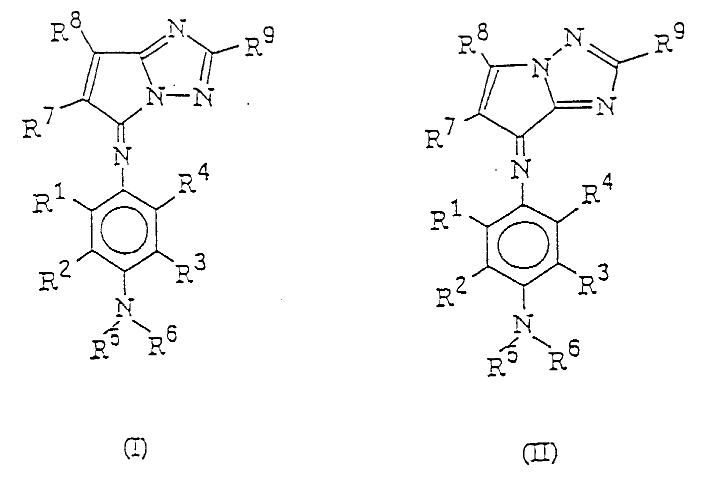 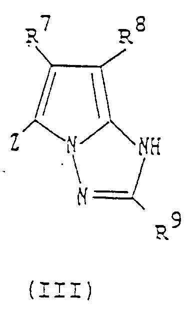 wherein:
R¹, R², R³, and R⁴ each independently represents a hydrogen atom, an alkyl group, an alkoxy group or an acylamino group;
R⁵ and R⁶ each independently represents a hydrogen atom, an alkyl group, an aryl group, or a heterocyclic group;
R⁷ represents a cyano group, an alkoxycarbonyl group, a trifluoromethyl group, or a carbamoyl group;
R⁸ represents a cyano group, a sulfonyl group, a carbamoyl group, an alkoxycarbonyl group, a trifluoromethyl group or an aryloxycarbonyl group;
R9 represents a hydrogen atom, an unsubstituted aryl group or an aryl group substituted by at least one of substituents selected from a nitro group, a halogen atom, a cyano group, an acylamino group, and a sulfonamido group, a heterocyclic group, an alkyl group, a cyano group, a carboxyl group, a formyl group, an acyl group, a carbamoyl group, an alkoxycarbonyl group, an aryloxycarbonyl group, an acylamino group, an alkoxycarbonylamino group, an aryloxycarbonylamino group, a sulfonylamino group, an aminocarbonylamino group, a sulfamoylamino group, an amino group, an alkoxy group, an aryloxy group, a silyloxy group, a heteryloxy group, an alkylthio group, an arylthio group, a heterylthio group, a halogen atom, a hydroxyl group, a nitro group, a sulfamoyl group, a sulfonyl group, an azo group, an acyloxy group, a carbamoyloxy group, an imido group, a sulfinyl group, a phosphoryl group, or an azolyl group;
the sum of Hammett's substituents constant σₚ of R⁷ and R⁸ is 0.65 or more;
and
Z represents a hydrogen atom, a halogen atom, an arylthio group, a heterocyclic thio group, an arylsulfinyl group, or a nitroso group, wherein the arylthiogroup is an unsubstituted arylthio group or an arylthio group substituted by at least one of substituents selected from a halogen atom, an alkyl group, an alkoxy group, a cyano group, aryloxycarbonyl group, an alkoxycarbonyl group, an ureido group,
an alkoxycarbonylamino group, an acylamino group and a carboxylgroup, and the arylsulfinyl group is an unsubstituted aryl sulfinyl group or an aryl sulfinyl group substituted by at least one of substituents selected from a halogen atom, an alkyl group an alkoxy group, a cyano group, an alkoxycarbonyl group and a carboxyl group.

2. A pyrrolotriazole derivative as in claim 1, wherein said pyrrolotriazole derivative is a pyrrolotriazole azomethine dye represented by any one of formulae (I) or (II).

3. A pyrrolotriazole derivative as in claim 1, wherein said pyrrolotriazole derivative is a 1H-pyrrolo- [1,2-b] [1,2,4] -triazole derivative represented by formula (III).

4. A pyrrolotriazole derivative as in claim 1, wherein R², R³ and R⁴ each represents a hydrogen atom; and R¹ represents a hydrogen atom, an alkyl group, or an acylamino group.

5. A pyrrolotriazole derivative as in claim 1, wherein R⁷ is a carbamoyl group, an alkoxycarbonyl group or a cyano group.

6. A pyrrolotriazole derivative as in claim 1, wherein R⁸ represents a cyano group or -COR, wherein R represents an amino group, an alkoxy group, or an aryloxy group.

7. A pyrrolotriazole derivative as in claim 1, wherin R⁹ represents an unsubstituted aryl group or an aryl group substituted by at least one of substituents selected from a nitro group, a halogen atom, a cyano group, an acylamino group, and a sulfonamido group.

8. A pyrrolotriazole derivative as in claim 3, wherin R⁷ represents a cyano group or a -COR group, wherein R represents an amino group or an alkoxy group, and R⁸ represents a cyano group or a -COR group, wherein R represents an amino group, an alkoxy group, or an aryloxy group; and R⁹ represents an unsubstituted aryl group or an aryl group substituted by at least one of substituents selected from a nitro group, a halogen atom, a cyano group, an acylamino group, and a sulfonamido group.

9. A pyrrolotriazole derivative as in claim 8, wherein Z represents a hydrogen atom; a halogen atom; an unsubstituted arylthio group; an arylthio group substituted by at least one of substitutents selected from a halogen atom, an alkyl group, an alkoxy group, a cyano group, an aryloxcarbonyl group, an alkoxycarbonyl group, a ureido group, an alkoxycarbonylamino group, an acylamino group, and a carboxyl group; an unsubstituted arylsulfinlyl group; or an arylsulfinyl group substituted by at least one of substitutents selected from a halogen atom, an alkyl group, an alkoxy group, a cyano group, an alkoxycarbonyl group, and a carboxyl group.

## Patentansprüche

1. Pyrrolotriazolderivat, dargestellt durch irgendeine der Formeln (I), (II) oder (III): 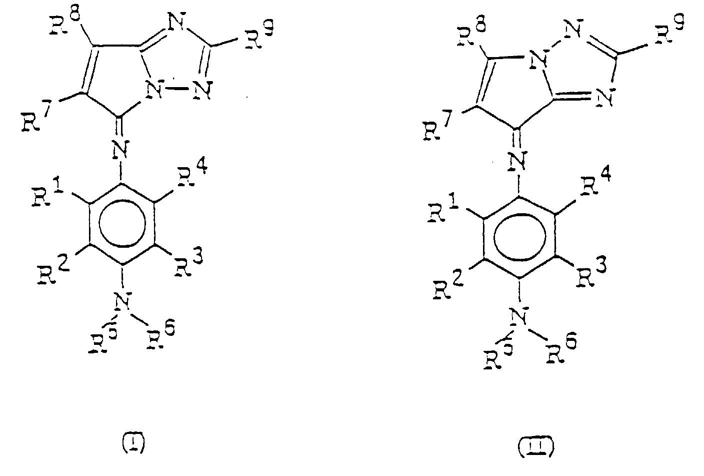 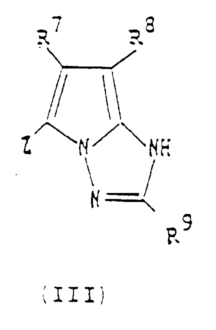 worin
R¹, R², R³ und R⁴ jeweils unabhängig ein Wasserstoffatom, eine Alkylgruppe, eine Alkoxygruppe oder eine Acylaminogruppe darstellen;
R⁵ und R⁶ jeweils unabhängig ein Wasserstoffatom, eine Alkygruppe, eine Arylgruppe oder eine heterocyclische Gruppe darstellen;
R⁷ eine Cyanogruppe, eine Alkoxycarbonylgruppe, eine Trifluoromethylgruppe oder eine Carbamoylgruppe darstellt;
R⁸ eine Cyanogruppe, eine Sulfonylgruppe, eine Carbamoylgruppe, eine Alkoxycarbonylgruppe, eine Trifluoromethylgruppe oder eine Aryloxycarbonylgruppe darstellt;
R⁹ ein Wasserstoffatom, eine unsubstituierte Arylgruppe oder eine Arylgruppe substituiert mit mindestens einem der Substituenten ausgewählt unter einer Nitrogruppe, einem Halogenatom, einer Cyanogruppe, einer Acylaminogruppe, und einer Sulfonamidogruppe; eine heterocyclische Gruppe, eine Alkylgruppe, eine Cyanogruppe, eine Carboxylgruppe, eine Formylgruppe, eine Acylgrupe, eine Carbamoylgruppe, eine Alkoxycarbonylgruppe, eine Aryloxycarbonylgruppe, eine Acylaminogruppe, eine Alkoxycarbonylaminogruppe, eine Aryloxycarbonylaminogruppe, eine Sulfonylaminogruppe, eine Aminocarbonylaminogruppe, eine Sulfamoylaminogruppe, eine Aminogruppe, eine Alkoxygruppe, eine Aryloxygruppe, eine Silyloxygruppe, eine Heteryloxygruppe, eine Alkylthiogruppe, eine Arylthiogruppe, eine Heterylthiogruppe, ein Halogenatom, eine Hydroxylgruppe, eine Nitrogruppe, eine Sulfamoylgruppe, eine Sulfonylgruppe, eine Azogruppe, eine Acyloxygruppe, eine Carbamoyloxygruppe, eine Imidogruppe, eine Sulfinylgruppe, eine Phosphorylgruppe oder eine Azolylgruppe darstellt;
die Summe der Hammett's Substituentenkonstanten σₚ von R⁷ und R⁸ 0,65 oder mehr beträgt; und
Z ein Wasserstoffatom, ein Halogenatom, eine Arylthiogruppe, eine heterocyclische Thiogruppe, eine Arylsulfinylgruppe oder eine Nitrosegruppe darstellt, wobei die Arylthiogruppe eine unsubstituierte Arylthiogruppe oder eine Arylthiogruppe substituiert mit mindestens einem der Substituenten ausgewählt unter einem Halogenatom, einer Alkylgruppe, einer Alkoxygruppe, einer Cyanogruppe, einer Aryloxycarbonylgruppe, einer Alkoxycarbonylgruppe, einer Ureidogruppe, einer Alkoxycarbonylaminogruppe, einer Acylaminogruppe und einer Carboxylgruppe ist und die Arylsulfinylgruppe eine nicht substituierte Arylsulfinylgruppe oder eine Arylsulfinylgruppe substituiert durch mindestens einen der Substituenten ausgewählt unter einem Halogenatom, einer Alkylgruppe, einer Alkoxygruppe, einer Cyanogruppe, einer Alkoxycarbonylgruppe und einer Carboxylgruppe ist.

2. Das Pyrrolotriazolderivat nach Anspruch 1, worin besagtes Pyrrolotriazolderivat ein Pyrrolotriazolazometinfarbstoff ist, dargestellt durch irgendeine der Formeln (I) oder (II).

3. Das Pyrrolotriazolderivat nach Anspruch 1, worin besagtes Pyrrolotriazolderivat ein 1H-Pyrrolo-[1,2-b] [1,2,4]-triazolderivat ist, dargestellt durch Formel (III).

4. Das Pyrrolotriazolderivat nach Anspruch 1, worin R², R³ und R⁴ jeweils ein Wasserstoffatom darstellen und R¹ ein Wasserstoffatom, eine Alkylgruppe oder eine Acylaminogruppe darstellt.

5. Das Pyrrolotriazolderivat nach Anspruch 1, worin R⁷ eine Carbamoylgruppe, eine Alkoxycarbonylgruppe oder eine Cyanogruppe ist.

6. Das Pyrrolotriazolderivat nach Anspruch 1, worin R⁸ eine Cyanogruppe oder -COR darstellt, worin R eine Aminogruppe, eine Alkoxygruppe oder eine Aryloxygruppe darstellt.

7. Das Pyrrolotriazolderivat nach Anspruch 1, worin R⁹ eine nicht substituierte Arylgruppe oder eine Arylgruppe substituiert mit mindestens einem der Substituenten ausgewählt unter einer Nitrogruppe, einem Halogenatom, einer Cyanogruppe, einer Acylaminogruppe und einer Sulfonamidogruppe darstellt.

8. Das Pyrrolotriazolderivat nach Anspruch 3, worin R⁷ eine Cyanogruppe oder eine -COR Gruppe darstellt, worin R eine Aminogruppe oder eine Alkoxygruppe darstellt; R⁸ eine Cyanogruppe oder eine -COR Gruppe darstellt, worin R eine Aminogruppe, eine Alkoxygruppe oder eine Aryloxygruppe darstellt; und R⁹ eine unsubtituierte Arylgruppe oder eine Arylgruppe substituiert durch mindestens einen der Substituenten ausgewählt unter einer Nitrogruppe, einem Halogenatom, einer Cyanogruppe, einer Acylaminogruppe und einer Sulfonamidogruppe darstellt.

9. Das Pyrrolotriazolderivat nach Anspruch 8, worin Z ein Wasserstoffatom, ein Halogenatom, eine unsubstituierte Arylthiogruppe, eine Arylthiogruppe substituiert durch mindestens einen der Substituenten ausgewählt unter einem Halogenatom, einer Alkylgruppe, einer Alkoxygruppe, einer Cyanogruppe, einer Aryloxycarbonylgruppe, einer Alkoxycarbonylgruppe, einer Ureidogruppe, einer Alkoxycarbonylaminogruppe, einer Acylaminogruppe und einer Carboxylgruppe; eine unsubtituierte Arylsulfinylgruppe oder eine Arylsulfinylgruppe substituiert durch mindestens einen der Substituenten ausgewählt unter einem Halogenatom, einer Alkylgruppe, einer Alkoxygruppe, einer Cyanogruppe, einer Alkoxycarbonylgruppe und einer Carboxylgruppe darstellt.

## Revendications

1. Dérivé de pyrrolotriazole représenté par l'une quelconque des formules (I), (II) ou (III): 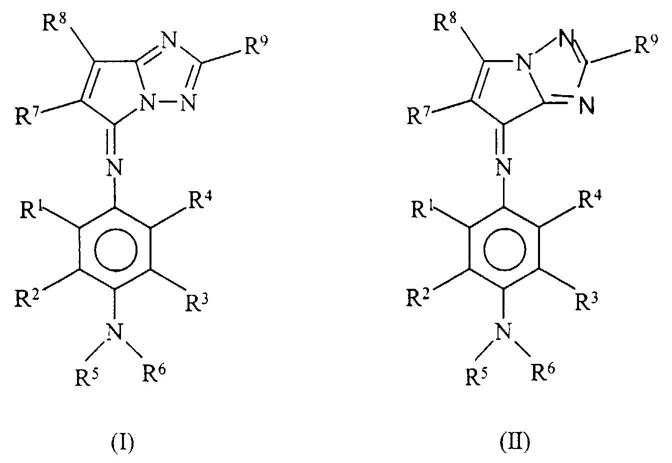 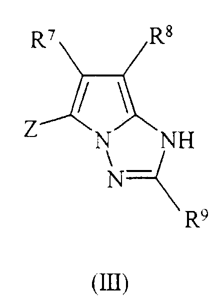 dans lesquelles:
R¹, R², R³ et R⁴ représentent chacun indépendamment un atome d'hydrogène, un groupe alkyle, un groupe alcoxy ou un groupe acylamino;
R⁵ et R⁶ représentent indépendamment un atome d'hydrogène, un groupe alkyle, un groupe aryle ou un groupe hétérocyclique ;
R⁷ représente un groupe cyano, un groupe alcoxycarbonyle, un groupe trifluorométhyle ou un groupe carbamoyle;
R⁸ représente un groupe cyano, un groupe sulfonyle, un groupe carbamoyle, un groupe alcoxycarbonyle, un groupe trifluorométhyle ou un groupe aryloxycarbonyle;
R⁹ représente un atome d'hydrogène, un groupe aryle non substitué ou un groupe aryle substitué par au moins un substituant choisi parmi un groupe nitro, un atome d'halogène, un groupe cyano, un groupe acylamino, et un groupe sulfonamido, un groupe hétérocyclique, un groupe alkyle, un groupe cyano, un groupe carboxyle, un groupe formyle, un groupe acyle, un groupe carbamoyle, un groupe alcoxycarbonyle, un groupe aryloxycarbonyle, un groupe acylamino, un groupe alcoxycarbonylamino, un groupe aryloxycarbonylamino, un groupe sulfonylamino, un groupe aminocarbonylamino, un groupe sulfamoylamino, un groupe amino, un groupe alcoxy, un groupe aryloxy, un groupe silyloxy, un groupe hétéryloxy, un groupe alkylthio, un groupe arylthio, un groupe hétérylthio, un atome d'halogène, un groupe hydroxyle, un groupe nitro, un groupe sulfamoyle, un groupe sulfonyle, un groupe azo, un groupe acyloxy, un groupe carbamoyloxy, un groupe imido, un groupe sulfinyle, un groupe phosphoryle ou un groupe azolyle;
la somme des constantes des substituants de Hammett σₚ de R⁷ et de R⁸ est de 0,65 ou supérieure;
et
Z représente un atome d'hydrogène, un atome d'halogène, un groupe arylthio, un groupe thio hétérocyclique, un groupe arylsulfinyle ou un groupe nitroso, dans lequel le groupe arylthio est un groupe arylthio non substitué ou un groupe arylthio substitué par au moins un substituant choisi parmi un atome d'halogène, un groupe alkyle, un groupe alcoxy, un groupe cyano, un groupe aryloxycarbonyle, un groupe alcoxycarbonyle, un groupe uréido, un groupe alcoxycarbonylamino, un groupe acylamino et un groupe carboxyle, et le groupe arylsulfinyle est un groupe arylsulfinyle non substitué ou un groupe arylsulfinyle substitué par au moins un substituant choisi parmi un atome d'halogène, un groupe alkyle, un groupe alcoxy, un groupe cyano, un groupe alcoxycarbonyle et un groupe carboxyle.

2. Dérivé de pyrrolotriazole selon la revendication 1, dans lequel ledit dérivé de pyrrolotriazole est un colorant de pyrrolotriazole azométhine représenté par l'une quelconque des formules (I) ou (II).

3. Dérivé de pyrrolotriazole selon la revendication 1, dans lequel ledit dérivé de pyrrolotriazole est un dérivé de 1H-pyrrolo-[1,2-b][1,2,4]-triazole représenté par la formule (III).

4. Dérivé de pyrrolotriazole selon la revendication 1, dans lequel R², R³ et R⁴ représentent chacun un atome d'hydrogène ; et R¹ représente un atome d'hydrogène, un groupe alkyle ou un groupe acylamino.

5. Dérivé de pyrrolotriazole selon la revendication 1, dans lequel R⁷ est un groupe carbamoyle, un groupe alcoxycarbonyle ou un groupe cyano.

6. Dérivé de pyrrolotriazole selon la revendication 1, dans lequel R⁸ représente un groupe cyano ou -COR, dans lequel R représente un groupe amino, un groupe alcoxy ou un groupe aryloxy.

7. Dérivé de pyrrolotriazole selon la revendication 1, dans lequel R⁹ représente un groupe aryle non substitué ou un groupe aryle substitué par au moins un substituant choisi parmi un groupe nitro, un atome d'halogène, un groupe cyano, un groupe acylamino et un groupe sulfonamido.

8. Dérivé de pyrrolotriazole selon la revendication 3, dans lequel R⁷ représente un groupe cyano ou un groupe -COR, dans lequel R représente un groupe amino ou un groupe alcoxy et R⁸ représente un groupe cyano ou un groupe -COR, dans lequel R représente un groupe amino, un groupe alcoxy ou un groupe aryloxy ; et R⁹ représente un groupe aryle non substitué ou un groupe aryle substitué par au moins un substituant choisi parmi un groupe nitro, un atome d'halogène, un groupe cyano, un groupe acylamino et un groupe sulfonamido.

9. Dérivé de pyrrolotriazole selon la revendication 8, dans lequel Z représente un atome d'hydrogène ; un atome d'halogène ; un groupe arylthio non substitué ; un groupe arylthio substitué par au moins un substituant choisi parmi un atome d'halogène, un groupe alkyle, un groupe alcoxy, un groupe cyano, un groupe aryloxycarbonyle, un groupe alcoxycarbonyle, un groupe uréido, un groupe alcoxycarbonylamino, un groupe acylamino et un groupe carboxyle ; un groupe arylsulfinyle non substitué ; ou un groupe arylsulfinyle substitué par au moins un substituant choisi parmi un atome d'halogène, un groupe alkyle, un groupe alcoxy, un groupe cyano, un groupe alcoxycarbonyle et un groupe carboxyle.
